# FASCICULE DE BREVET EUROPEEN

(11) **EP 2 501 734 B1**
(45) Date de publication et mention de la délivrance du brevet: **02.09.2015**
(21) Numéro de dépôt: 10776749.3
(22) Date de dépôt: 16.11.2010
(51) Int. Cl.: C08F 220/60, C12N 11/06

(54) **POLYMÈRES COMPRENANT UNE MAJORITÉ DE MONOMÈRES AMPHIPHILES DESTINÉS AU PIÉGEAGE ET À LA MANIPULATION DE PROTÉINES MEMBRANAIRES**
POLYMERE MIT EINER MEHRHEIT AN AMPHIPHILEN MONOMEREN ZUM EINFANGEN UND ZUR MANIPULATION VON MEMBRANPROTEINEN
POLYMERS COMPRISING A MAJORITY OF AMPHIPHILIC MONOMERS INTENDED FOR TRAPPING AND MANIPULATING MEMBRANE PROTEINS

(30) Priorité: 16.11.2009 FR 0958072
(43) Date de publication de la demande: 26.09.2012
(73) Titulaire: Centre National de la Recherche Scientifique (CNRS), 75016 Paris (FR); Universite D'Avignon et Des Pays Du Vaucluse, 84029 Avignon Cedex 1 (FR)
(72) Inventeur: PUCCI, Bernard, F-13940 Mollégès (FR); POPOT, Jean-Luc, F-75013 Paris (FR); SHARMA, Kshatrapati Shivaji, Majra 248171 (IN); BAZZACCO, Paola, F-75017 Paris (FR); DURAND, Gregory, F-30400 Villeneuve Les Avignon (FR); GIUSTI, Fabrice, F-92330 Sceaux (FR)
(74) Mandataire: Regimbeau
(86) Numéro de dépôt international: PCT/EP2010/067609
(87) Numéro de publication internationale: WO 2011/058195

(56) Documents cités:
- WO-A1-90/10023
- FR-A1- 2 873 123
- SHIVAJI SHARMA K ET AL: "Glucose-Based amphiphilic telomers designed to keep membrane proteins soluble in aqueous solutions: synthesis and physicochemical characterization", LANGMUIR, AMERICAN CHEMICAL SOCIETY, WASHINGTON, DC, US LNKD- DOI:10.1021/LA8023056, vol. 24, no. 23, 1 janvier 2008 (2008-01-01), pages 13581-13590, XP007914140, ISSN: 0743-7463 [extrait le 2008-04-11] cité dans la demande

## Description

### DOMAINE DE L'INVENTION

L'invention concerne des polymères amphiphiles utiles pour la manipulation de composés hydrophobes en solution aqueuse, les complexes hydrosolubles formés entre des composés hydrophobes, en particulier des protéines membranaires, et ces polymères, des procédés de préparation de ces complexes, et les applications de ces complexes, notamment aux méthodes de diagnostic ou d'analyse.

### ART ANTERIEUR

Les protéines membranaires intégrales, une classe particulière de protéines, sont insérées *in vivo* dans les membranes biologiques dont elles traversent la double couche lipidique. La surface de ces protéines venant naturellement au contact des membranes (zone transmembranaire) est particulièrement hydrophobe, les surfaces extra-membranaires étant, elles, principalement hydrophiles. Les protéines membranaires assurent des fonctions biologiques essentielles, notamment en ce qui concerne les échanges d'information ou de molécules entre les divers compartiments cellulaires et entre la cellule et son environnement.

A ce titre, les protéines membranaires présentent un intérêt majeur dans le domaine médical. Elles représentent, par exemple, des cibles privilégiées pour les molécules médicamenteuses. Elles sont également impliquées dans de nombreuses maladies humaines, dont certaines (par exemple la sclérose en plaques ou la *myasthenia gravis*) ont une composante autoimmune manifestée par la présence dans le sérum d'autoanticorps dirigés contre des protéines membranaires.

La manipulation en solution aqueuse des protéines membranaires est le plus souvent un préalable indispensable à leur purification et à leur étude structurale et fonctionnelle. Elle nécessite d'éviter l'agrégation spontanée des domaines hydrophobes et, à cette fin, de maintenir autour des zones transmembranaires un environnement amphiphile.

Les préparations classiques de telles protéines à l'état hydrosoluble contiennent des concentrations supramicellaires de tensioactifs particuliers, les détergents. Le succès du procédé repose sur l'adsorption sur les régions protéiques transmembranaires de ces composés amphiphiles et dispersants. La manipulation des complexes ainsi formés est toutefois beaucoup plus délicate que celle des protéines solubles, en raison précisément de la présence de détergent. Celui-ci doit être présent à une concentration supérieure à sa concentration micellaire critique (cmc) dans toutes les solutions contenant la protéine étudiée. Outre les éventuels problèmes de coût posés par la consommation de détergent, les expériences sont souvent rendues délicates du fait que les protéines membranaires sont le plus souvent instables en solution détergente. Ainsi, en présence d'un excès de micelles, elles ont tendance à se dénaturer irréversiblement, tandis qu'un défaut de tensioactif conduit en général à leur précipitation.

Cette situation a conduit à rechercher des alternatives à l'utilisation des détergents, parmi lesquelles on citera par exemple les bicelles, qui sont de petits disques lipidiques stabilisés par des tensioactifs, les nanodisques, dont la structure est similaire mais où le tensioactif est une protéine, les peptitergents, qui sont des peptides amphiphiles, les lipopeptides, également peptidiques mais porteurs de chaînes hydrocarbonées, les tensioactifs fluorés ou hémifluorés, et les amphipols, à la famille desquels appartiennent les molécules faisant l'objet de la présente demande de brevet. Les amphipols sont des polymères amphiphiles spécialement conçus pour se substituer aux détergents à la surface transmembranaire des protéines membranaires (Tribet et al, WO 1998/027434). Ce brevet décrit l'emploi de copolymères amphiphiles pour le maintien des protéines membranaires en milieu aqueux.

La plupart des amphipols ou molécules prétendues telles décrits à ce jour sont des polymères ioniques, en particulier anioniques, ce qui interdit leur emploi dans divers systèmes analytiques (isoélectrofocalisation) ou de séparation (chromatographie sur colonne échangeuse d'ions), et n'est pas un facteur favorable à la cristallisation des protéines membranaires ainsi stabilisées. Il existe donc un besoin pour des polymères amphiphiles ayant les avantages des amphipols existants pour la manipulation des protéines membranaires et qui seraient non ioniques.

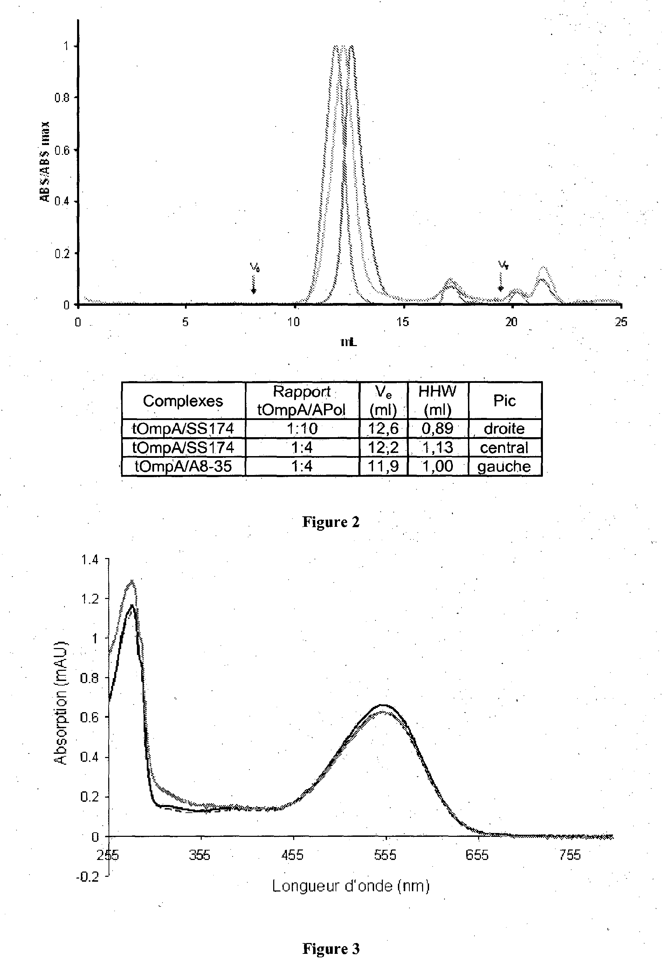

Des amphipols non ioniques ont été décrits dans Prata et al et Sharma et al.. Dans Prata et al, les amphipols sont des copolymères comprenant deux types de monomères (voir Figure 2 de ce document), l'un hydrophile (2 OH et 1 sucre ou 3 OH) et l'autre amphiphile (2 OH et une chaîne grasse). Dans ce document, le rapport molaire entre les monomères hydrophiles et les monomères amphiphiles a été maintenu entre 3,0 et 6,7, ce qui correspond à 75-87% de monomères hydrophiles et 13-25% de monomères amphiphiles, qui sont donc minoritaires.

Dans Sharma et al, les amphipols sont des copolymères comprenant deux types de monomères (voir Schéma 1 de ce document), l'un hydrophile (2 OH et 1 sucre) et l'autre amphiphile (1 OH, 1 sucre et une chaîne grasse). Dans ce document, le rapport molaire entre les monomères hydrophiles A et les monomères amphiphiles B a été maintenu entre 3 et 5, ce qui correspond à 75-83% de monomères hydrophiles A et 17-25% de monomères amphiphiles B, qui sont donc minoritaires. Cela s'explique par le fait que les auteurs ont constaté que l'amphipol comprenant le plus fort pourcentage (25%) de monomères amphiphiles avait déjà une solubilité aqueuse réduite.

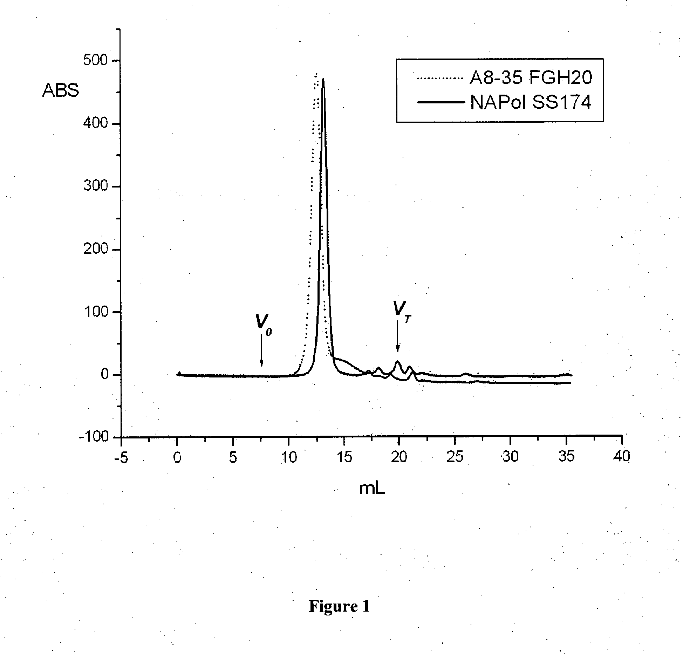

La demande WO 2008/058963 décrit l'immobilisation de protéines membranaires sur des supports à l'aide d'amphipols qui sont des copolymères comprenant différents types de monomères (hydrophiles, amphiphiles ou hydrophobes), dans lesquels le rapport du pourcentage total de monomères hydrophobes ou amphiphiles au pourcentage total de monomères hydrophiles est compris entre 0,25 et 2,5 (voir revendication 3 dé WO 2008/058963). L'amphipol exemplifié est un copolymère ionique comprenant des monomères hydrophiles et des monomères hydrophobes (voir Figure 1A de ce document). De plus, les groupements définis comme amphiphiles dans cette demande comprennent des fonctions hydrophiles et hydrophobes mélangées au sein du même "greffon", et non des groupes hydrophiles et des groupes hydrophobes distincts, greffés séparément sur la chaîne latérale.

Ainsi, tous les amphipols ou molécules prétendues telles décrits à ce jour sont des copolymères, comportant des unités de propriétés différentes, les unes hydrophiles, les autres hydrophobes et/ou amphiphiles, les monomères amphiphiles étant minoritaires lorsqu'ils sont présentes.

En outre, les résultats présentés dans Sharma et al suggèrent qu'il est nécessaire d'inclure des monomères hydrophiles en plus des monomères amphiphiles, afin de conserver une solubilité aqueuse suffisante des amphipols.

Cependant, la structure copolymérique de toutes les molécules décrites à ce jour présente un inconvénient important : elle rend difficile de reproduire exactement la même structure chimique d'un lot à l'autre. La synthèse en effet nécessite soit une copolymérisation radicalaire, soit une fonctionnalisation aléatoire d'un précurseur de type homopolymère, deux types de réactions non sélectives par essence. Il existe donc un besoin pour les polymères amphiphiles présentant les mêmes avantages pour la manipulation de composés hydrophobes, et des protéines membranaires en particulier, que ceux de l'art antérieur, et dont la préparation serait beaucoup plus reproductible d'un lot à l'autre.

### DESCRIPTION DE L'INVENTION

Contrairement à ce qui est suggéré dans Sharma et al (4), les inventeurs ont montré de façon surprenante que des homopolymères amphiphiles (homoAPols) constitués de monomères amphiphiles, ou des copolymères comprenant très majoritairement de tels monomères amphiphiles (« quasi-homopolymères ») peuvent avoir une solubilité dans l'eau suffisante pour permettre la manipulation des protéines membranaires aussi bien que les amphipols copolymériques connus dans l'art antérieur. En outre, ces homopolymères ou quasi-homopolymères peuvent être fabriqués de façon tout à fait reproductible et n'ont donc pas les inconvénients des amphipols copolymériques connus dans l'art antérieur.

La présente demande concerne donc un polymère amphiphile comprenant au moins 75%, au moins 80%, avantageusement au moins 85%, au moins 90%, au moins 95%, au moins 96%, au moins 97%, au moins 98%, au moins 99%, voire 100% de monomères amphiphiles de formule (I) : 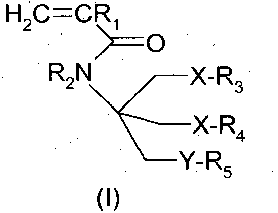 dans laquelle
R₁ et R₂ sont indépendamment choisis parmi H ou un groupe alkyle en C₁-C₃ (de préférence un méthyle) ;
X et Y sont indépendamment choisis parmi un atome d'oxygène, un atome de soufre, un groupe carbonyloxy (-(CO)O-) ou oxycarbonyl (-O(CO)-), un groupe uréthane (-OCONH-), et un groupe amide de formule (-CONR₆-) ou (-NR₆CO-) où R₆ est un atome d'hydrogène ou une alkyle en C₁-C₆ (de préférence un méthyle ou un éthyle) ;
R₃ et R₄ sont indépendamment choisis parmi :
   a) les groupements glycosidiques,
   b) les résidus zwiterioniques,
   c) les groupements poly(oxyalkylène) de formule -(O(CH₂)ₓ)_{y}-OH, où x est compris entre 1 et 6 (avantageusement x vaut 2) et y est compris entre 4 et 30, avantageusement entre 4 et 20 ou entre 4 et 10,
   d) les groupements alkylamides de formule -(CH₂)ₙCONR₇R₈ ou-(CH₂)ₙNR₇COR₈ où n est compris entre 1 et 4, R₇ et R₈ sont choisis indépendamment parmi un atome d'hydrogène (-H), un groupe alkyle en C₁-C₆ (de préférence un méthyle), un groupement glycosidique, un résidu zwitterionique ou un groupement poly(oxyalkylène) de formule -(O(CH₂)ₓ)_{y}-OH où x est compris entre 1 et 6 (avantageusement x vaut 2) et y est compris entre 4 et 30, avantageusement entre 4 et 20 ou entre 4 et 10,
R₅ est une chaîne hydrocarbonée cyclique (R5 peut contenir un ou deux cycles saturés ou non, notamment de type cyclohexane, cyclopentane ou aromatique) ou acyclique (linéaire ou ramifiée), saturée ou insaturée (une ou plusieurs insaturations) comprenant de 5 à 16 atomes de carbones, ou une chaîne hémifluorocarbonée de formule CₜF₂ₜ₊₁(CH₂)ₘ avec t compris entre 2 et 10 et m compris entre 2 et 10.
la masse molaire moyenne du polymère étant comprise entre 800 et 100 000, ce qui correspond à un nombre de monomères compris entre 1 et 120, avantageusement inférieure ou égale à 50 000, de préférence entre 8000 et 50000. La masse molaire moyenne est donnée en poids.

On entend par « alkyle en Cx-Cy » un radical hydrocarboné saturé linéaire ou ramifié de formule -CⱼH₂ⱼ₊₁, où x ≤ j ≤ y. Notamment, un alkyle en C₁-C₆ peut être un alkyle en C₁ (méthyle), C₂ (éthyle), C₃ (n-propyle, ou isopropyle), C₄ (n-butyle, isobutyle, sec-butyle ou tert-butyle), C₅ (ex : n-pentyle, néopentyle, isopentyle, tert-pentyle) ou C₆ (n-hexyle par exemple).

Lorsque le polymère selon l'invention comprend d'autres monomères que ceux de formule (I), ces monomères sont des monomères à motif acrylique ou vinylique avec une chaîne latérale substituée par un groupement hydrophile ou hydrophobe. En particulier, les groupements hydrophiles ou hydrophobes des chaînes latérales peuvent être choisis parmi ceux définis dans les revendications 3 et 4 de la publication PCT WO 2008/058963, le contenu de ces revendications étant incorporé par référence.

Avantageusement, R₁ et R₂ sont indépendamment choisis parmi H ou un groupe méthyle. Plus avantageusement, R₁ et/ou R₂ sont un atome d'hydrogène.

Avantageusement aussi, X est un atome d'oxygène.

Avantageusement aussi, Y est un groupe uréthane (-OCONH-).

Par « groupement glycosidique », on entend tout groupement comprenant un sucre. Les groupements glycosidiques avantageux pour R₃ et/ou R₄ sont notamment :
- les mono- ou di-saccharides, ou
- les mono- ou di-saccharides aminés,

Par « monosaccharide » ou « ose », on entend un monomère de glucide non hydrolysable. Avantageusement, le monosaccharide est choisi parmi les hexoses (oses à 6 atomes de carbone), notamment parmi le glucose, mannose, galactose, aliose, altrose, idose, ou maltose.

Par « disaccharide » ou « diholoside », on entend un sucre formé par deux oses liés par une liaison osidique hydrolysable par voie chimique (emploi d'acides concentrés à chaud) ou par voie enzymatique. Avantageusement, le disaccharide est choisi parmi les dihexoses, formés de deux hexoses, tels que le lactose (Galactose β(1→4) Glucose), le cellobiose (Glucose β(1→4) Glucose) ou le maltose (Glucose α (1->4) Glucose).

Par « polysaccharide », on entend un sucre constitué d'un polymère linéaire ou branché, composé d'au moins 2 monomères choisis parmi les monosaccharides tels que définis précédemment et pouvant atteindre 20 unités, tels que certains amyloses. Le terme polysaccharide inclut donc les disaccharides (ou dioses), les trisaccharides (ou trioses), etc... jusqu'à 20 unités monosaccharides. De préférence, les unités monosacharides sont des unités hexoses tels que définis précédemment.

Par « mono-, di-, ou poly-saccharide aminé » on entend tout monosaccharide, disaccharide ou polysaccharide tel que défini précédemment dont une ou plusieurs fonctions alcool (-OH) a/ont été substituée(s) par une aminé (-NH₂). On peut notamment citer comme exemples de monosaccharides aminés la glucosamine, la galactosamine, la fructosamine, ou la mannosamine, et comme exemple de di-saccharide aminé l'aminolactitol.

Les mono- ou di-saccharides, notamment sont préférés, en particulier les mono-ou di-hexoses de type glucose, mannose, galactose, lactose, allose, altrose, idose, lactose, maltose, ou cellobiose; le glucose, le mannose et le galactose étant particulièrement préférés, surtout le glucose.

Ces groupements glycosidiques sont greffés, en particulier lorsque X est un atome d'oxygène, soit par l'oxygène du carbone anomère (O glycosylation), soit par celui de l'hydroxyle primaire (liaison ester) soit par la fonction aminé (liaison amide), soit enfin par le groupement azoture dont on aura préalablement doté le carbone anomère en substitution du groupe hydroxyle. Dans ce dernier cas les sucres sont introduits par l'intermédiaire de là réaction de Huygens sur un motif propargyle préalablement greffé sur la fonction X qui en l'occurrence sera un atome d'oxygène. Avantageusement, le groupement glycosidique est greffé par l'oxygène du carbone anomère (O glycosylation)

Par « résidu zwitterionique », on entend un groupement possédant des charges électriques formelles d'une unité, de signes opposés et situées en général sur des atomes non adjacents. Ces composés possédant en même temps des charges positives et négatives, ils sont très solubles dans l'eau, qui est un solvant polaire. Les résidus zwiterioniques avantageux sont issus par exemple de bétaines simples (notamment de type -N⁺(CH₃)₂C(CH₂)ᵢCO₂⁻, -N⁺(CH₃)₂C(CH₂)ᵢSO₃⁻ N⁺(CH₃)₂C(CH₂)ᵢOSO₃⁻ avec i compris entre 1 et 10), ou de motifs acide aminés, notamment tels que la lysine, l'ornithine, l'acide aspartique ou glutamique dotés d'un groupement acrylique polymérisable tels que CH₂=CHCONH-(CH₂)ⱼ- avec j compris entre 2 et 5.

Dans un mode de réalisation avantageux, R₃ et/ou R₄ sont un groupement glycosidique, de préférence un monosaccharide ou disaccharide ou un mono- ou di-saccharide aminé, avantageusement un mono- ou di-saccharide. De préférence, le mono-ou di-saccharide est un mono- ou di-hexose, notamment de type type glucose, mannose, galactose, lactose, allose, altrose, idose, lactose, maltose, ou cellobiose, avantageusement un glucose, un mannose ou un galactose, encore de préférence un glucose.

Avantageusement, R₅ est une chaîne hydrocarbonée cyclique (R5 peut contenir un ou deux cycles saturés ou non, notamment de type cyclohexane ou cyclopentane) ou acyclique (linéaire ou ramifiée), saturée ou insaturée (une ou plusieurs insaturations), avantageusement linéaire et/ou saturée, comprenant de 5 à 16 atomes de carbones. De préférence, R₅ est un groupe alkyle en C₅-C₁₆, de préférence en C₈-C₁₂, notamment en C₁₁, avantageusement linéaire.

Plus précisément, un polymère avantageux selon l'invention comprend au moins 75%, de préférence au moins 80%, avantageusement au moins 85%, au moins 90%, au moins 95%, au moins 96%, au moins 97%, au moins 98%, au moins 99%, voire 100% de monomères amphiphiles de formule (II) : 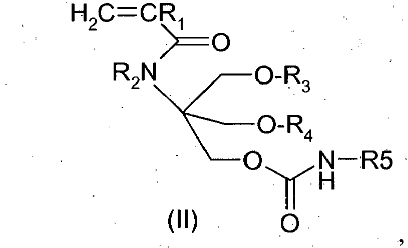 dans laquelle
R₁ et R₂ sont indépendamment choisis parmi H ou un groupe alkyle en C₁-C₆ (de préférence méthyle),
R₃ et R₄ sont des groupements glycosidiques tels que définis précédemment,
R₅ est une chaîne hydrocarbonée cyclique (R5 peut contenir un ou deux cycles saturés ou non, notamment de type cyclohexane, ou cyclopentane ou aromatique) ou acyclique (linéaire ou ramifiée), saturée ou insaturée (une ou plusieurs insaturations) comprenant de 5 à 16 atomes de carbones telle que définie précédemment.

Avantageusement, R₃ et R₄, identiques ou différents, de préférence identiques, sont des mono- ou di-saccharides, de préférence des mono- ou di-hexoses, notamment de type glucose, mannose, galactose, lactose, allose, altrose, idose, lactose, maltose, ou cellobiose, de préférence un glucose, un mannose ou un galactose, avantageusement R₃ et R₄ sont des glucoses.

Avantageusement, R₅ est un alkyle en C₅-C₁₆, de préférence en C₈-C₁₂, notamment en C₁₁, de préférence linéaire.

Dans un mode de réalisation avantageux :
- R₃ et R₄ sont identiques et représentent un glucose, un mannose ou un galactose, de préférence un glucose, et
- R₅ est un alkyle en C₅-C₁₆, de préférence en C₈-C₁₂, notamment en C₁₁, avantageusement linéaire.

Un polymère particulièrement avantageux selon l'invention comprend au moins 75%, de préférence au moins 80%, avantageusement au moins 85%, au moins 90%, au moins 95%, au moins 96%, au moins 97%, au moins 98%, au moins 99%, voire 100% de monomères amphiphiles de formule (III) :

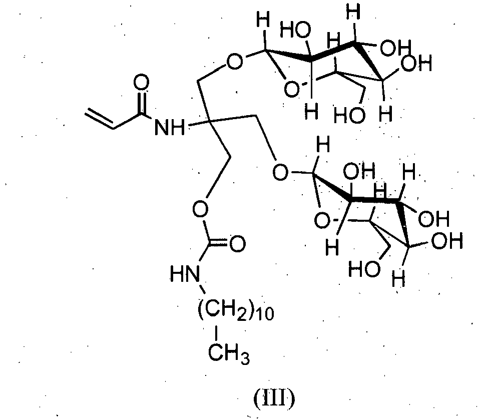

Les monomères de formule (I), (II) ou (III) telle que décrite précédemment sont des monomères à motif acrylique ou vinylique comprenant une chaîne grasse hydrophobe et deux groupements hydrophiles (groupements glycosidiques ou résidus zwiterioniques). Ils sont synthétisables par des réactions chimiques bien connues des spécialistes telles que des réactions de glycosylation, d'amidification ou par l'usage d'isocyanate. La synthèse du monomère de formule (III) est décrite en détails dans les exemples. Une voie de synthèse tout à fait comparable peut être utilisée pour greffer des galactoses ou mannoses à la place des glucoses, et/ou pour greffer un autre type de chaîne grasse, notamment toute autre chaîne alkyle.

Le polymère selon l'invention comprend majoritairement des monomères amphiphiles. Dans un mode de réalisation avantageux, le polymère selon l'invention est un homopolymère comprenant 100% de monomères de formule (I), (II), ou (III) telle que définie ci-dessus formant une chaîne homogène, éventuellement liée en tête de chaîne à un autre groupement.

En effet, les polymères selon l'invention peuvent être préparés par polymérisation amorcée par des initiateurs radicalaires tels que l'AIBN ou le peroxyde de benzoyle dans des solvants anhydres portés à un minimum de 60°C tels que le THF, l'acétonitrile ou encore le méthanol, le solvant préféré étant le THF. Avantageusement, la taille du polymère lors de sa synthèse est contrôlée par ajout d'agent de transfert de chaîne de type thiol, le rapport des concentrations de ce dernier et du monomère contrôlant la taille du polymère. Le deuxième intérêt de la présence de cet agent de transfert est d'autoriser l'introduction en extrémité de chaîne d'un groupement spécifique susceptible d'être utilisé pour ses propriétés particulières. Ainsi, dans ce cas, le polymère selon l'invention comprend un groupement spécifique en tête de chaîne du polymère. Lorsqu'il est fait référence à un homopolymère selon l'invention, cela inclut donc la possibilité de la présence en tête de chaîne du polymère d'un groupement spécifique distinct provenant de l'agent de transfert de chaîne, et qui a pu ensuite être modifié.

Notamment, le polymère selon l'invention peut en outre comprendre en tête de chaîne (c'est-à-dire à l'une de ses extrémités) un groupement comprenant une fonction thiol de formule R₉-S-, où R₉ est avantageusement choisi parmi :
- (CH₂)ₘ COOH avec m = 1 à 11,
- (CH₂)ₘ-NH₂ avec m = 2 à 11,
- (CH₂)ₘ-X-R₁₀ avec m = 1 à 11 ; X = O, NH, COO, CONH, S, phosphonate P(O)(O-R₁₀)₂; et R₁₀ choisi parmi H, CH₃, un groupe benzoyle ou benzyle, un agent fluorescent (tel que le NBD, un dérivé de la fluorescéine ou de la rhodamine...) une biotine, un polysaccharide (notamment un trisaccharide) linéaire ou ramifié comprenant des hexoses, un agent piégeur de radicaux libres tel qu'une nitrone ou une espèce paramagnétique cyclique de type nitroxyde.

- (CH₂)ₘ-CONH(CH₂)ₚS-R₁₁ avec m compris entre 1 et 10, p compris entre 2 et 11, et R₁₁ choisi parmi H, -C(C₆H₅)₃, un agent fluorescent tel que le NBD ou la fluorescéine, un agent piégeur de radicaux libres tel qu'une nitrone ou une espèce paramagnétique cyclique de type nitroxyde., un oligomère dérivé d'un monomère acrylique ou vinylique tel que l'acrylate de méthyle, l'acrylamide, le THAM, l'acetate de vinyle.
- (CH₂)ₘ-CO(OCH₂CH₂)ₓOCO(CH₂)ₚS-R₁₁ avec m compris entre 1 et 10, x compris entre 3 et 100, p compris entre 2 et 11, et R₁₁ est tel que défini ci-dessus,
- (CH₂)₂-(-OCH₂CH₂)_{q}-O-R₁₀ avec q = 1 à 100, et R₁₀ est tel que défini ci-dessus,
- (CH₂)ᵣCONHC(CH₂OR₁₂)₃, -CH₂CONHC(CH₃)(CH₂OR₁₂)₂, ou-CH₂CONHCH(CH₂OR₁₂)₂ avec r compris entre 1 et 11, et R₁₂ est choisi parmi H, un groupe benzyle ou un groupe benzoyle, un agent fluorescent (tel que le NBD, un dérivé de la fluorescéine ou de la rhodamine), une biotine, un monosaccharide ou un polysaccharide linéaire ou branché, éventuellement aminé, de préférence composé de monomères de mannose, galactose, glucose, acide sialique, glucosamine, galactosamine, et/ou mannosamine, un agent piégeur de radicaux libres tel qu'une nitrone ou une espèce paramagnétique cyclique de type nitroxyde.
- (CH₂)ₘ P(O)(OR₁₃)₂ avec m compris entre 2 et 11, et R₁₃ représente un groupement alkyle de C₁ à C₁₆ linéaire éventuellement substitué,
- une chaîne hydrocarbonée linéaire comprenant 3 à 20 atomes de carbones, saturée ou insaturée, éventuellement substituée en particulier par un ou plusieurs groupes OH, avantageusement un groupe alkyle linéaire en C₃-C₂₀ ou alcényle linéaire en C₃-C₂₀ éventuellement substitué par un ou plusieurs groupes OH (comme le phytol par exemple), ou
- une chaîne perfluorée de formule CₜF₂ₜ₊₁ (CH₂)ₘ avec t compris entre 2 et 10 et m compris entre 2 et 10.

L'ensemble de ces composés de type thiol est soit accessible commercialement soit aisément préparé par des réactions chimiques simples à haut rendement.

Par « alcényle linéaire en C₃-C₂₀ », on entend une chaîne hydrocarbonée linéaire comprenant 3 à 20 atomes de carbones et comportant au moins une double liaison.

Avantageusement, R₉ représente -(CH₂)ᵣCONHC(CH₂OR₁₂)₃,-CH₂CONHC(CH₃)(CH₂OR₁₂)₂, ou -CH₂CONHCH(CH₂OR₁₂)₂ avec r compris entre 1 et 11, où R₉ représente H, un groupe benzyle ou un groupe benzoyle, un agent fluorescent (tel que le NBD, un dérivé de la fluoresceine ou de la rhodamine), une biotine, ou un monosaccharide ou un polysaccharide linéaire ou branché, éventuellement aminé, de préférence composé de monomères de mannose, galactose, glucoe, acide sialique, glucosamine, galactosamine, et/ou mannosamine.

Un agent de transfert de chaîne particulièrement préféré est celui où R₉ est - (CH₂)₂CONHC(CH₂OH)₃.

Dans le cas où le polymère selon l'invention comprend 100% de monomères de formule (I), cela résulte alors en un polymère de formule (IV) : 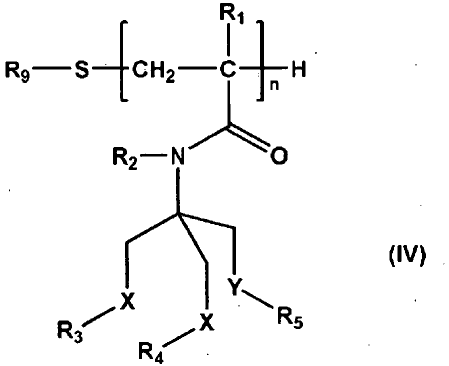 dans laquelle R₁ à R₅ et R₉ sont tels que définis précédemment, et n est tel que le polymère possède une masse molaire moyenne comprise entre 8000 et 100 000, ce qui correspond à n compris entre 10 et 120, avantageusement inférieure ou égale à 50 000 (n inférieur ou égal à 60), de préférence entre 8000 et 50000 (n compris entre 10 et 60).

Un polymère selon l'invention tout particulièrement préféré est de formule (V) : 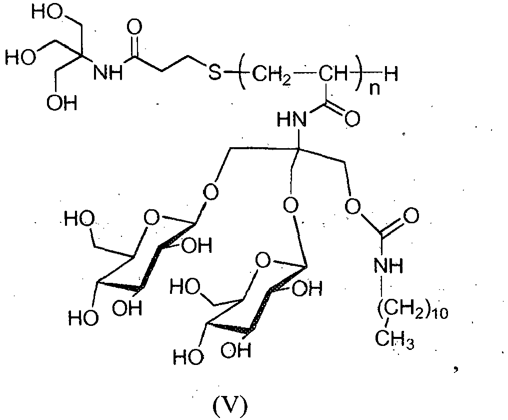 où n est compris entre 1 et 120, de préférence entre 1 et 60.

L'invention concerne également un procédé de préparation d'un polymère amphiphile selon l'invention, comprenant la réaction d'un monomère de formule (I), (II) ou (III) telle que décrite précédemment avec un agent de transfert de chaîne en présence d'un initiateur radicalaire dans un solvant anhydre à au moins 60°C.

L'agent de transfert de chaîne est un composé de type thiol, de préférence de formule (VI) :
R₉-SH (VI), dans laquelle R₉ est tel que défini précédemment.

L'initiateur radicalaire peut notamment être l'azobisisobutyronitrile (AIBN) ou le peroxyde de benzoyle.

L'invention concerne également un complexe hydrosoluble d'un composé hydrophobe ou amphiphile, avantageusement une protéine membranaire, et d'un polymère amphiphile selon l'invention. Avantageusement, la protéine membranaire est choisie dans le groupe constitué par les enzymes membranaires, les récepteurs membranaires, les canaux ioniques membranaires, les antigènes membranaires de microorganismes ou de tumeurs, et les protéines médicaments (telles que notamment les anticorps). Le complexe selon l'invention peut en outre se présenter sous forme congelée ou lyophilisée.

L'invention concerne aussi une solution aqueuse possédant une concentration supérieure à 1 g/l, avantageusement supérieure à 2 g/l, 3 g/l, ou 4 g/l, de préférence supérieure à 5 g/l, 6 g/l, 7 g/l, 8 g/l, 9 g/l, ou 10 g/l d'un ou plusieurs complexe(s) selon l'invention. La concentration est avantageusment inférieure à 500 g/l. De préférence la concentration de la solution est entre 10 et 500 g/l.

L'invention concerne également un produit comprenant un support et au moins un complexe selon l'invention, ledit complexe étant fixé sur ledit support par l'intermédiaire du polymère amphiphile selon l'invention.

L'invention concerne enfin l'utilisation d'un complexe, d'une solution aqueuse ou d'un produit selon l'invention pour détecter la présence ou l'absence dans un échantillon biologique d'un ligand dudit composé hydrophobe ou amphiphile.

### DESCRIPTION DES FIGURES

**Figure 1****. Estimation par filtration sur tamis moléculaire de la taille et de la dispersité des particules de télomère amphiphile.** Cent µL d'une solution-stock d'homotélomère (lot SS174) ont été dilués dans 900 µL de tampon Tris/HCl (20 mM Tris, 100 mM NaCl, pH = 8,5) et injectés sur une colonne de Superose 12 10-300GL. L'élution a été effectuée avec le tampon Tris et la détection à 220 nm. *V*₀ et *V*_{T} indiquent respectivement le volume exclu et le volume total de la colonne (respectivement 7.53 et 19.9 mL). Le rayon de Stokes apparent est de 2,6 nm. Pour comparaison, un échantillon d'amphipol anionique classique de type A8-35 a été analysé dans les mêmes conditions (lot FGH20). Le rayon de Stokes apparent des particules d'A8-35 est de 3,15 nm.
**Figure 2****. Estimation par filtration sur tamis moléculaire de la taille et de la dispersité des complexes tOmpA/télomère amphiphile**. Le domaine transmembranaire (tOmpA) de la protéine OmpA de la membrane externe de la bactérie *Escherichia coli* a été piégé à l'aidé d'un homotélomère amphiphile à deux rapports massiques protéine/polymère différents, 1:4 (pic central) ou 1:10 (pic de droite) et les échantillons dilués dans du tampon Tris/HCl (20 mM Tris, 100 mM NaCl, pH = 8,5) injectés sur une colonne de Superose 12 10-300GL. L'élution a été effectuée avec le tampon Tris et la détection à 280 nm. Les pics ont été normalisés au même maximum. *V*₀ et *V*_{T} indiquent respectivement le volume exclu et le volume total de la colonne. Pour comparaison, un échantillon de tOmpA piégé avec l'amphipol anionique classique de type A8-35 a été analysé dans les mêmes conditions (pic de gauche). Les volumes d'élution sont, de gauche à droite, de 11,9, 12,2 et 12, 6 mL ; les largeurs de pic à mihauteur sont respectivement de 1,00, 1,13 et 0,89 mL.
**Figure 3****. Spectre d'absorption UV/visible de la bactériorhodopsine après piégeage avec l'A8-35 ou avec des homotélomères non-ioniques.** La BR a été piégée à un rapport en masse protéine/amphipol de 1:5, l'amphipol étant soit l'A8-35 (lot FGH20 ; en noir), soit un homotélomère non-ionique (lot SS174 : en gris ; lot SS298 : en tirets noirs). Les spectres ont été enregistrés juste après l'élimination du détergent (2 h d'incubation à 4°C avec des BioBeads, centrifugation à 16.000 × g pendant 30 min).

### EXEMPLES

### EXEMPLE 1. Préparation des homopolymères amphiphiles

### 1.1. Synthèse du monomère acrylamide diglucosylé : Exemple du N-(1,1-di(-O-β-D-glucopyranosyloxyméthyl)-1-(undecylcarbamoyloxyméthyl)méthyl)acrylamide.

Selon un premier procédé, qui est celui ayant généré les monomères ayant été utilisés dans la suite des exemples, la synthèse se déroule en trois étapes à partir du THAM commercial (qui peut être obtenu avec un rendement supérieur à 90% à partir du Tris-(hydroxyméthyl)aminométhane), selon le schéma 1 suivant.

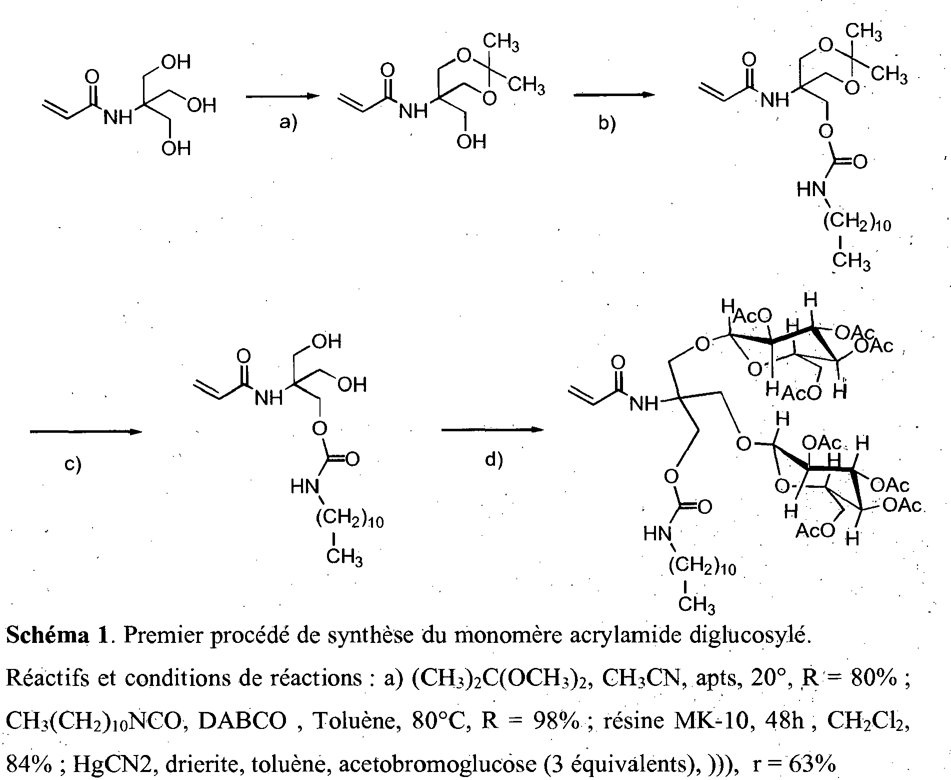

### Synthèse du THAM isopropylidène

Dans un premier temps, on bloque deux fonctions hydroxyles sous la forme d'un groupe isopropylidène en traitant pendant 24 h le THAM par du diméthoxypropane en présence d'une quantité catalytique d'acide paratoluène sulfonique dans l'acétonitrile à la température ambiante. Après traitement usuel, le THAM isopropylidène cristallise et est isolé avec un rendement de 80%.

### 5-acrylamido-5-undecylcarbamoyloxyméthyl-2,2-diméthyl-cyclo 1,3 dioxahexane

Le THAM isopropylidène (2.64 g, 12.28 mmol, 1.0 equiv.) et le 1,4-diaza bicyclo[2,2,2]octane DABCO (4.05 g, 14.74 mmol, 1.2 equiv.) sont dissous dans du toluene anhydre et le mélange est chauffé à reflux pendant 30mn sous argon. Le dodecyl isocyanate (2.91 g, 14.74 mmol, 1.2 equiv.) en solution dans le toluène est ajouté goutte à goutte à la solution maintenue à 80°C. Après 12H d'agitation, 5 gouttes de méthanol sont ajoutés et le mélange jeté dans de l'acétate d'éthyle (150 mL). La phase organique Test lavée avec HCl 1N (3 × 100 mL) et une solution saturée de NaCl (2 × 100 mL), séchée sur Na₂SO₄ et concentrée sous vide pour conduire au composé THAM isopropylidène doté d'une chaîne undecyle liée par un groupement carbamate (5.0 g, 12.12 mmol, 98%) sous forme de poudre blanche. R_{f} ∼ 0.7, éthylacetate/cyclohexane (7:3 v/v). ¹H NMR (CDCl₃ δ 7.01 (s, 1H), 6.21 (dd, *J* = 1.6 and 17.0 Hz, 1H), 6.08 (dd, *J =* 10.0 and 17.0 Hz, 1H), 5.65 (dd, *J* = 1.6 and 10.0 Hz, 1H), 4.99 (m, 1H), 4.72 (d, *J* = 12.1 Hz, 2H), 3.62 (d, *J =* 12.0 Hz, 2H), 3.20 (q, *J =* 6.7 Hz, 2H), 1.62 (s, 3H), 1.48 (m, 2H), 1.42 (s, 3H), 1.27 (s, 18H), 0.89 (t, *J* = 6.6 Hz, 3H). ¹³C NMR (CDCl₃ δ 165.7, 157.6 (CO), 131.4 (CH), 126.5 (CH₂), 98.5 (C), 64.9, 60.5 (CH₂), 53.5 (C), 43.0, 41.3, 31.9, 31.3, 29.8, 29.6, 29.6, 29.5, 29.3, 26.7 (CH₂), 26.6 (CH₃), 22.7 (CH₂), 21.0, 14.1 (CH₃).

### N-(1,1-(2',3',4',6'tetra-O-acetyl-β-D-glucopyranosyloxy-méthyl)-1-(undecylacarbamoyloxyméthyl)-méthyl)-acryl-amide

Le composé précédent (5.0 g, 12.12 mmol) et de la résine MK-10 (30 g) sont agités dans du dichloromethane (200 mL) pendant 48 h, la résine est ensuite filtré sur une courte colonne de célite et rincée au méthanol (2 × 100 mL). La phase organique est concentrée sous vide pour conduire au *N*-(1,1-bishydroxyméthyl-1-(undecylcarbamoyloxyméthyl) méthyl)-acrylamide (3.8 g, 10.2 mmol, 84%). Ce composé (2.0 g, 5.37 mmol, 1.0 equiv.), du cyanure de mercure (2.13 g, 16.10 mmol, 3.0 equiv.) et de la drierite sont mélangés dans du toluene sous argon. Après 2 minutes de sonication, le Bromotetraacetylglucose TAGB (6.62 g, 16.10 mmol, 3 equiv.) est ajouté et le mélange soumis à sonication pendant 30 mn. Le mélange réactionnel est ensuite filtré sur Célite et rincé à l'acétate d'éthyle (100 mL). Les phases organiques sont lavées successivement avec une solution saturée de bicarbonate de sodium (2 × 100 mL), eau (100 mL), solution à 10% iodure de potassium (4 × 50 mL), solution saturée de thiosulphate thiosulphate (4 × 50 mL) et eau (2 × 50 mL). Les phases organiques sont séchées sur Na₂SO₄ et concentrées sous pression réduite, le brut résultant est soumis à une chromatographie flash, éluée avec éthylacetate/cyclohexane (3:7 v/v) pour conduire au monomère attendu sous forme d'une poudre blanche. (3.5 g, 3.39 mmol, 63 %). Rf ∼ 0.35, éthylacetate/ cyclohexane (7:3 v/v). Mp 58.0°C. [α_{D}²⁵] = -12.70 (c, 1, CH₂Cl₂). ¹H NMR (CDCl₃) δ 6.92 (s, 1H), 6.24 (dd, *J* = 1.4 and 16.0 Hz, 1H), 6.04 (dd, *J =* 10.0 and 16.9 Hz, 1H), 5.64 (dd, *J* = 1.4 and 10.0 Hz, 1H), 5.3-4.9 (m, 7H), 4.5 (m, 2H), 4.4-3.9 (m, 10H), 3.71 (dt, *J* = 2.4 and 7.3 Hz, 2H), 3.16 (q, *J* = 6.5 Hz, 2H), 2.11, 2.09, 2.07, 2.05, 2.02 (5s, 24H), 1.34 (m, 18H), 0.89 (t, *J* = 6.6 Hz, 3H). ¹³C NMR (CDCl₃) δ 170.8, 170.7, 170.7, 170.2, 169.6, 169.5, 169.5, 169.5, 165.7, 157.2 (CO), 131.3 (CH), 126.6 (CH₂), 101.0, 100.8, 77.3, 72:6, 72.5, 71.8, 71.8, 71.1, 68.3, 68.2, (CH), 68.6, 68.3, 68.0, 64.5, 61.7, 60.4 (CH₂), 59.6 (C), 41.2, 31.9, 29.8, 29.6, 29.3, 26.9, 26.8, 26.8, 22.7 (CH₂), 21.1, 20.8, 20.8, 20.7, 20.7, 20.6, 20.6, 20.6, 14.2 (CH₃). HRMS (ESI+) calcd for C₄₇H₇₂N₂O₂₃ ([M + H]+): 1033.4599 Found: 1033.4609 [M+H]+.

Selon un procédé alternatif, la synthèse du monomère acrylamide (*N*-(1,1-di(-*O-β*-*D*-glucopyranosyloxyméthyl)-1(undecylcarbamoyloxyméthyl)méthyl)acrylamide) se déroule en deux étapes à partir du THAM commercial (qui peut être obtenu avec un rendement supérieur à 90% à partir du Tris-(hydroxyméthyl)aminométhane), selon le schéma 1 suivant.

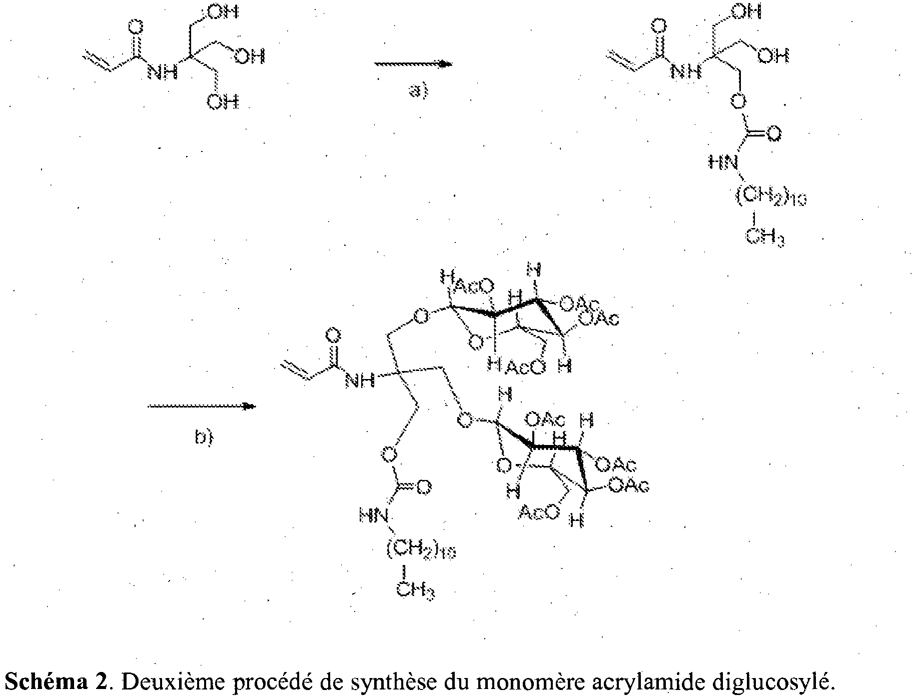

Réactifs et conditions de réactions : a) THAM (5 equiv), CH₃(CH₂)₁₀NCO (1 equiv.), DABCO (0.5 equiv), DMF, 60°C, 3H, R = 80%; b) HgCN₂, drierite, toluène, acetobromoglucose (3 équivalents), ))), r = 63%

### N-1,1-di(hydroxymethylmethyl)-1(undecylcarbamoyloxymethyl)-methyl) acrylamide

A une solution agitée de THAM (21.9 g, 125 mMol, 5 equiv) et de Diazabicyclo[2,2,2]octane (DABCO) (1.5 g, 13.4 mMol, 0.5 equiv) dans 40 mL de DMF chauffée à 60°C, est ajouté goutte à goutte sous atmosphère d'argon l'undecyl isocyanate (5g, 25 mMol, 1 equiv) préalablement solubilisé dans 10 mL de chlorure de méthylène. Le mélange réactionnel est maintenu à 60°C jusqu'à totale disparition de l'undecylisocyanate (∼30 mn). Les solvants sont ensuite évaporés sous pression réduite et le précipité repris avec 200 mL de chlorure de méthylène. La suspension est agitée mécaniquement 15 mn à température ambiante. Le précipité résiduel est filtré et remis à nouveau en suspension dans 100 mL de chlorure de méthylène puis filtré à nouveau. L'opération est reproduite deux fois. Le précipité restant est immédiatement recristallisé dans le méthanol anhydre pour donner 16.5 g de THAM qui peuvent ainsi être remis en réaction. Les phases organiques sont jointes, lavées avec 2x50 mL d'une solution de HCl 1N, 2x50 mL d'une solution saturée de carbonate de sodium et 2x50 mL d'eau, séchées sur sulphate de sodium et concentrées sous pression réduite. Le produit brut est cristallisé dans une solution de AcOEt/Hexane 2/8 pour donner le N-1,1-di(hydroxymethylmethyl)-1(undecylcarbamoyloxymethyl)-methyl)acrylamide sous forme de poudre blanche (7.55 g, R = 80%). Rf ∼ 0.5 (ethylacetate/cyclohexane (8:2 v/v) ¹H NMR (DMSO*d₆*) δ 7.56 (s, 1H), 7.12 (t, *J* = 5, 1H), 6.36 (dd, *J* = 10 and 17.5Hz, 1H), 6.04 (dd, *J* = 2.2 and 17.5 Hz, 1H), 5.56 (dd, *J* = 2.2 and 10 Hz, 1H), 4.87 (m, 2H), 4.17, (s, 2H), 3.63 (s, 2H), 3.61 (s, 2H), 2.95 (m, 2H), 1.37 (m, 2H), 1.24 ( m, 16H), 0.86 (t, J= 6.75, 3H). ¹³C NMR (DMSO*d₆*) δ 166.5, 156.8, 132.8, 125.5 (CO), 62.6, 61.5, 60.4 (CH₂), 31.8, 29.9, 29.5, 29.2, 29.3, 26.7, 22.6, 14.5 (CH₃).

### N-(1,1-(2',3',4',6'tetra-O-acetyl-β-D-glucopyanosyloxy-méthyl)-1-(undecylcarbamoyloxyméthyl)-méthyl)-acryl-amide

Le composé précédent (2.0 g, 5.37 mmol, 1.0 equiy.), du cyanure de mercure (2.13 g, 16.10 mmol, 3.0 equiv.) et de la drierite sont mélangés dans du toluene sous argon. Après 2 minutes de sonication, le Bromotetraacetylglucose TAGB (6.62 g, 16.10 mmol, 3 equiv.) est ajouté et le mélange soumis à sonication pendant 30 mn. Le mélange réactionnel est ensuite filtré sur Célite et rincé à l'acétate d'éthyle (100 mL). Les phases organiques sont lavées successivement avec une solution saturée de bicarbonate de sodium (2 × 100 mL), eau (100 mL), solution à 10% iodure de potassium (4 × 50 mL), solution saturée de thiosulphate (4 × 50 mL) et eau (2 × 50 mL). Les phases organiques sont séchées sur Na₂SO₄ et concentrées sous pression réduite, le brut résultant est soumis à une chromatographie flash, éluée avec éthylacetate/cyclohexane (3:7 v/v) pour conduire au monomère attendu sous forme d'une poudre blanche. (3.5 g, 3.39 mmol, 63 %). Rf ∼ 0.35, éthylacetate/ cyclohexane (7:3 v/v). Mp 58.0°C. [α_{D}²⁵] = -12.70 (c, 1, CH₂Cl₂). ¹H NMR (CDCl₃) δ 6.92 (s, 1H), 6.24 (dd, *J* = 1.4 and 16.0 Hz, 1H), 6.04 (dd, *J* = 10.0 and 16.9 Hz, 1H), 5.64 (dd, *J* = 1.4 and 10.0 Hz, 1H), 5.3-4.9 (m, 7H), 4.5 (m, 2H), 4.4-3.9 (m, 10H), 3.71 (dt, *J =* 2.4 and 7.3 Hz, 2H), 3.16 (q, *J* = 6.5 Hz, 2H), 2.11, 2.09, 2.07, 2.05, 2.02 (5s, 24H), 1.34 (m, 18H), 0.89 (t, *J* = 6.6 Hz, 3H). ¹³C NMR (CDCl₃) δ 170.8, 170.7, 170.7, 170.2, 169.6, 169.5, 169.5, 169.5, 165.7, 157.2 (CO), 131.3 (CH), 126.6 (CH₂), 101.0, 100.8, 77.3, 72.6, 72.5, 71.8, 71.8, 71.1, 68.3, 68.2, (CH), 68.6, 68.3, 68.0, 64.5, 61.7, 60.4 (CH₂), 59.6 (C), 41.2, 31.9, 29.8, 29.6, 29.3, 26.9, 26.8, 26.8, 22.7 (CH₂), 21.1, 20.8, 20.8, 20.7, 20.7, 20.6, 20.6, 20.6, 14.2 (CH₃). HRMS (ESI+) calcd for C₄₇H₇₂N₂O₂₃ ([M + H]+): 1033.4599 Found: 1033.4609 [M+H]+.

### 1.2. Synthèse du NAPol.

La synthèse du télomère (Schéma 3) s'appuie sur l'utilisation d'un agent de transfert dérivé de l'acide mercaptopropionique doté d'un groupement Tris polybenzoylé. Ces différents groupes benzoyles présentent une forte absorption UV et se trouveront à l'extrémité de la chaîne du polymère. De ce fait, ils permettent la détermination exacte, par mesure de l'absorption UV du produit final, de la masse du télomère et donc du degré de polymérisation moyen. Il faut noter ici que le choix du motif tribenzoylé peut être pris comme un exemple des possibilités d'introduction (par l'intermédiaire de la nature de l'agent de transfert utilisé) de motifs intéressants (fluorescéine, cholestérol, biotine, nitrones... etc.) et donc de la fonctionnalisation de l'extrémité de chaîne. Cette fonctionnalisation peut également avoir lieu après télomérisation par l'intermédiaire de groupements de type ester actif (tels que hydroxysuccinimide, paranitro-benzoate, pentafluoro-benzoate...etc..) préalablement introduits sur le télogène

La synthèse du NAPol est résumée sur le schéma 3 suivant:

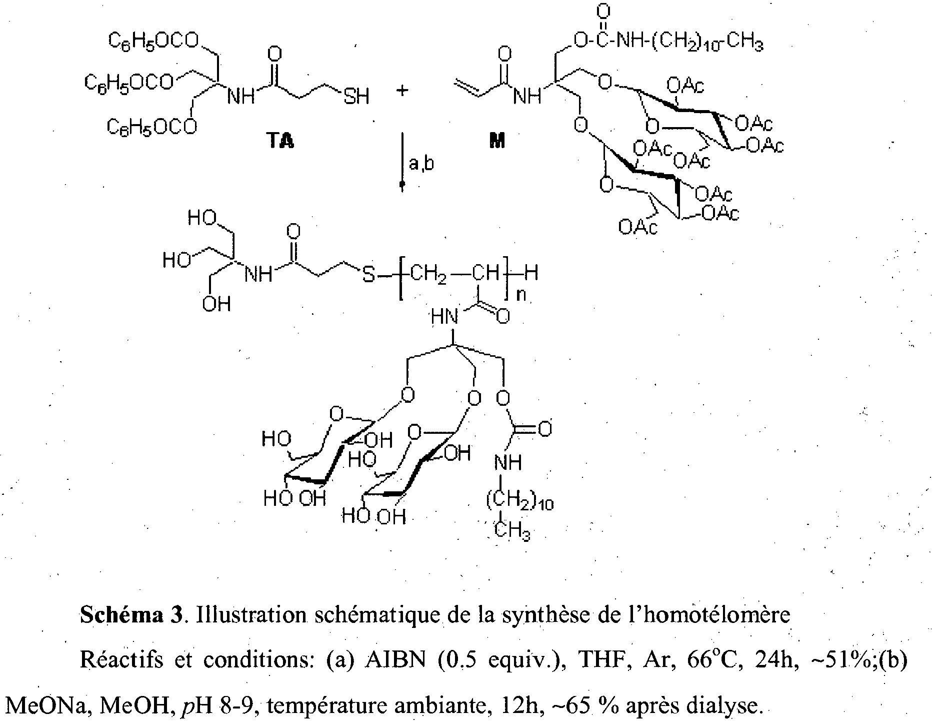

Le monomère THAM, N-(1,1-(2',3',4',6'tetra-O-acetyl-β-D-glucopyranosyloxy-méthyl)-1-(undecylcarbamoyloxyméthyl)-méthyl)-acryl-amide (1.0 g, 0. 968 mmol, 40.0 equiv.) est dissous dans le THF (15 ml). La solution est dégazée par bullage d'argon et chauffage à reflux pendant 30 mn. L'agent télogène TA (12.62 mg, 0.024 mmol, 1.0 equiv.) dont la synthèse a été précédemment décrite (Sharma et al) et l'AIBN (1.98 mg, 0.012 mmol, 0.5 equiv.) dissous dans le THF sont alors ajoutés avec une microseringue. Le mélange réactionnel est agité à reflux jusqu'à disparition totale dû monomère (∼24 h). Il est ensuite concentré sous vide, et le télomère brut est isolé par chromatographie d'exclusion de taille (Sephadex® LH-20) en éluant avec un mélange MeOH/CH₂Cl₂ (1:1, v/v), puis séché sous vide. Le télomère sous forme protégée est isolé sous forme de poudre blanche (0.524 g, 52%). R*_{f}* = 0.0 éthylacetate/cyclohexane (6:4 v/v). ¹H NMR (250 MHz, CDCl₃, δ ppm) 0.8 (-C*H*₃ de la chaîne alkyle), 1.3-1.7 (-*CH*₂)₁₀ de la chaîne alkyle), 2.1-2.4 (broad s, -OCOCH₃), 3.1 (-N*H*- méthylène vicinal), 4.8- 5.3 (m, unité glucose *2H, 3H, 4H, 5H,* et *6H*), 6.6 (-N*H*), 7.4-8.1 (trois t, C₆*H*₅ du TA).

Après détermination de la masse moléculaire par RMN ¹H et UV, l'homotélomère (2.0 g, 1.91 mmol) est dissous dans du méthanol anhydre (50 mL) sous atmosphère d'Argon. Une quantité catalytique de Methoxide de sodium MeONa est ajouté et la solution agitée à la température ambiante pendant une nuit. La solution est ensuite neutralisée avec de la résine IRC 50 acide (jusqu'à pH = 8) par agitation pendant 15 mn. Après filtration de la résine et évaporation du solvant, le télomère est soumis à une dialyse avec une membrane dont le seuil de coupure est de 6-8 KDa Le polymère purifié est isolé par lyophilisation, il est obtenu sous forme de poudre blanche (0.850 mg, 65%). ¹H NMR (250 MHz, DMSO-*d*₆, δ ppm) 0.8 (-C*H*₃ de la chaîne alkyle), 1.2-1.6 (-C*H*₂))₁₀ de la chaîne alkyle), 3.2 (-N*H* méthylene vicinal), 4.8- 5.2 (m, unité glucose *2H, 3H, 4H, 5H,* et *6H*), 7.1 (-N*H*).

La technique mise au point est universelle et peut et a déjà été appliquée à des motifs monomères porteurs de sucre différents (galactose et mannose en particulier) et a des monomères porteurs de chaînes fluorocarbonées. Elle peut par ailleurs être aisément étendue à des cotélomères incorporant divers types de monomères, qu'ils soient amphiphiles, hydrophobes ou hydrophiles, ainsi que nous l'avons établi précédemment pour des mélanges de monomères hydrophiles et hydrophobes.

### EXEMPLE 2. Reproductibilité de l'homotélomère obtenu

Différents homotélomères ont été synthétisés, en fonction des quantités relatives de monomère et d'agent télogène TA. Les conditions de synthèse et la structure chimique des homotélomères synthétisés sont résumées dans le Tableau 1.

**Tableau 1. Conditions de synthèse et structure chimique des différents NAPols**

| NAPol | Homotélomère | | Masse Mw moyenne /10³ ± 1x10³(g.mol⁻¹) | |
|---|---|---|---|---|
| | *Ro^{a}* | *DPn^{b}* | Protégé | Déprotégé |
| SS174 | 20 | 14 | 15 ±1 | 10 ±1 |
| SS293 | 20 | 11 | 12 ±1 | 8 ± 1 |
| SS298 | 40 | 42 | 44.0 ± 1 | 29 ± 1 |
| SS292 | 100 | 90 | 93 ± 2 | 63 ± 2 |
| SS325 | 15 | 16 | 17 ± 2 | 11,3 ± 0,5 |

| | | | | |
|---|---|---|---|---|
| *^{a}Rapport molaire initial Monomère*/*TA, ^{b.c}* Estimé par analyse UV | | | | |

De plus, SS298 et SS325 ont été synthétisés par plusieurs lots différents avec les mêmes précurseurs et les mêmes conditions. L'analyse de la reproductibilité des lots de SS298 est montrée dans le Tableau 2 suivant.

**Tableau 2. Reproductibilité des, lots de SS298**

| Lots | Homotélomère | | Télomère isolé après Sephadex LH 20 (mg) | Masse Moléculaire Doyenne sous forme acétylé déterminée par UV M_{w}/10³ (g. mol⁻¹) |
|---|---|---|---|---|
| | *Ro*^{a} | *DPn^{b}* | | |
| SS291 | 40 | 34 | 478 | 36 |
| SS294 | 40 | 46 | 532 | 48 |
| SS295 | 40 | 44 | 524 | 46 |
| SS296 | 40 | 55 | 534 | 57 |
| Combinés SS298 | 40 | 42 | - | 44 |

| | | | | |
|---|---|---|---|---|
| *^{a}Rapport molaire initial Monomère*/*TA, ^{b.c}* Estimé par analyse UV | | | | |

Les résultats montrent une bonne reproductibilité des lots d'homotélomère amphiphile selon l'invention.

### EXEMPLE 3. Caractérisation de l'homotélomère et propriétés physico-chimiques

Dans les exemples 3 et 4, les nouveaux homotélomères amphiphiles selon l'invention sont comparé à l'amphipol de référence A8-35, qui est un amphipol anionique copolymérique de formule :

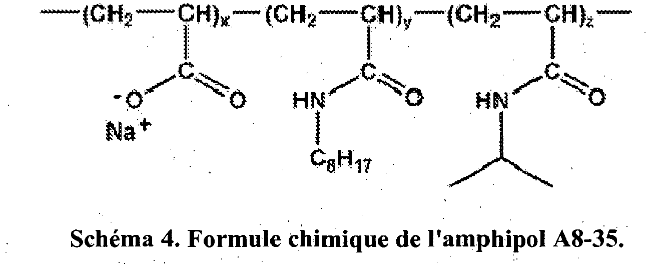

Tous les homotélomères amphiphiles selon l'invention préparés dans l'exemple 1 présentent, après hydrolyse des fonctions esters, une solubilité dans l'eau supérieure à 100 g/L. Les solutions sont incolores et moussent un peu après agitation vigoureuse. Aucune concentration micellaire critique (CMC) ni concentration d'agrégation critique (CAC) ne peut être détectée par mesure de tension de surface, ce qui indique que, comme pour l'amphipol de référence A8-35, la CAC est extrêmement basse. Des mesures de diffusion de neutrons aux petits angles (SANS; non montrées) et de filtration sur tamis moléculaire (SEC ; Fig. 1) indiquent que ce type de télomère s'associe en solution aqueuse pour donner des particules d'une masse totale de l'ordre de 50 kDa, ce qui correspond sensiblement à l'association de deux molécules de télomère et est proche des valeurs déterminées précédemment pour les amphipols classiques de type A8-35 (∼40 kDa). Leur rayon apparent est comparable à celui des particules d'A8-35 (∼2,6 *vs.* ∼3,15 nm), ainsi que leur dispersité (Fig. 1). Observées en diffusion quasi-élastique de la lumière (QLS), les solutions de ces télomères apparaissent formées de particules de taille homogène de 5-6 nm de diamètre, en bon accord avec les données de SEC (Tableau 3). La taille des particules est peu sensible à la concentration (Tableau 3) ou à la température (Tableau 4).

**Tableau 3. Diamètre des particules d'homotélomère non-ionique SS174 à des concentrations de 10, 50 et 100 g/L, déterminée par QLS à différentes températures.**

| Sample | Concentration (gL⁻¹) | *D_{H}* (nm) | Largeur du pic a mi hauteur (nm) | Distribution volumique du pic principal (en %) |
|---|---|---|---|---|
| SS174 | 10 | 5.8 | 1.4 | 100 |
| | 50 | 5.9 | 1.5 | 100 |
| | 100 | 6.3 | 1.6 | 100 |

**Tableau 4. Diamètre des particules d'homotélomère non-ionique SS298, SS293 et SS292 à une concentration de 50 g/L, déterminée par QLS à différentes températures.**

| Temp. (°C) | SS298 | | | SS293 | | | SS292 | | |
|---|---|---|---|---|---|---|---|---|---|
| | *D_{H}* (nm) | Largeur du pic a mi hauteur (nm) | Distribution volumiqtie du pic principal (en %) | *D_{H}* (nm) | Largeur du pic a mi hauteur (nm) | Distribution volumique du pic principal (en %) | *D_{H}* (nm) | Largeur du pic a mi hauteur (nm) | Distribution volumique du pic principal (en %) |
| 2 | 6.14 | 1.77 | 100 | 5.06 | 1.52 | 100 | 6.63 | 1.48 | 99.9 |
| 10 | 5.92 | 1.68 | 100 | 4.91 | 1.47 | 100 | 6.44 | 1.45 | 99.9 |
| 20 | 5.74 | 1.74 | 100 | 4.81 | 1.45 | 100 | 6.0 | 1.69 | 100 |
| 30 | 5.71 | 1.74 | 100 | 4.82 | 1.45 | 100 | 5.93 | 1.71 | 100 |
| 40 | 5.70 | 1.74 | 100 | 4.79 | 1.42 | 100 | 5.92 | 1.69 | 100 |
| 50 | 5.62 | 1.73 | 100 | 4.74 | 1.43 | 100 | 5.9 | 1.76 | 100 |
| 60 | 5.48 | 1.76 | 100 | 4.70 | 1.44 | 100 | a | a | a |
| 70 | 5.47 | 1.79 | 100 | a | a | a | 6.08 | 1.80 | 100 |

| | | | | | | | | | |
|---|---|---|---|---|---|---|---|---|---|
| *^{a}* non déterminé | | | | | | | | | |

Ainsi, la caractérisation des propriétés physico-chimiques des homotélomères amphiphiles non-ioniques selon l'invention montre que ces amphipols ont des propriétés similaires à celles de l'amphipol copolymérique anionique A8-35.

### EXEMPLE 4. Complexation de l'homotélomère avec les protéines membranaires

Les amphipols sont, par définition, des polymères àmphiphiles conçus pour garder les protéines membranaires solubles et biochimiquement stables en l'absence de détergents. La capacité des homotélomères non-ioniques selon l'invention à remplir ces deux fonctions a été testée sur deux protéines, la région transmembranaire de la protéine OmpA de la membrane externe d*'Eschenichia coli* (tOmpA) et la bactériorhodopsine (BR). Ces deux protéines sont représentatives des deux grands types de structures adoptées par les protéines transmembranaires, le tonneaux (tOmpA) et le faisceau d'hélices α (BR). Par ailleurs, la BR est une protéine relativement instable en solution détergente, et dont la dénaturation est aisément mesurée par la libération de son cofacteur, le rétinal, laquelle se traduit par une perte d'absorption vers 564 nm (disparition de l'holoprotéine) et l'apparition d'un pic à 380 nm (dû au rétinal libre).

Les données rassemblées dans le Tableau 5 indiquent que les deux lots d'homotélomères testés sont pratiquement aussi efficaces que l'amphipol anionique de référence A8-35 pour garder en solution les deux protéines après que la concentration du détergent ait été abaissée sous sa concentration micellaire critique soit par dilution avec du tampon sans détergent (tOmpA), soit par adsorption sur des billes de polystyrène (BR) : les taux de rétention en solution varient de 75 à 94 %, contre 89-98 % après complexation par l'A8-35, la différence observée pour tOmpA (∼75% *vs*. ∼90%) étant très probablement due à la densité plus importante des complexes formés avec les amphipols non-ioniques, qui cause une légère précipitation durant la centrifugation à haute vitesse utilisée comme test de maintien en solution. Une vitesse plus faible a été utilisée pour la BR, ce qui explique que la différence de maintien en solution soit moins importante (et la précipitation de la protéine en l'absence d'amphipol - ligne 2 - moins complète).

| **Expérience** | **APols** | **Rapport en poids MP/APol** | **tOmpA dans le surnageant** | **BR native dans le surnageant** |
|---|---|---|---|---|
| 1 | Aucun | 1:0 ([détergent] > CMC) | 98% | 85% |
| 2 | Aucun | 1:0 ([détergent] < CMC) | 5% | 17% |
| 3 | A8-35 | 1:4-1:5 | 89% | 98% |
| 4 | SS174 | 1:4-1:5 | 76% | 93% |
| 5 | SS174 | 1:10 | 75% | 92% |
| 6 | SS298 | 1:5 | n.d. | 93% |
| 7 | SS298 | 1:10 | n.d. | 94% |

**Tableau 5. Capacité des télomères non-ioniques à maintenir en solution les protéines membranaires.** Des solutions détergentes de tOmpA et de BR ont été additionnées d'homotélomère non-ionique du type décrit sur le schéma 3 ci-dessus, soit du lot SS174 (4-5), soit du lot SS298 (6-7), aux rapports en masse indiqués. Après 20 min d'incubation, les solutions de tOmpA ont été diluées avec du tampon sans détergent de façon à faire tomber la concentration de détergent sous la cmc, tandis que celles de BR étaient additionnées de billes de polystyrène (BioBeads), sur lesquelles le détergent s'adsorbe. Après 2 h d'incubation, les solutions ont été centrifugées 30 min à 200.000 × g (tOmpA) ou à 16.000 × g (BR). La fraction de protéine présente dans le surnageant a été estimée par mesure de l'absorption à 280 nm (tOmpA, BR) ou à 554 nm (BR). Les contrôles incluent la dilution des échantillons de protéine en solution détergente avec une solution de détergent au dessus de la cmc de celui-ci (1), ou avec du tampon sans détergent (2), et une expérience de piégeage avec l'amphipol anionique A8-35 (3), réalisée dans les mêmes conditions que les expériences 4-7. n.d. : non déterminé.

Des données préliminaires (non montrées) indiquent que les complexes BR/télomères non-ioniques sont d'une taille comparable (en SEC) aux complexes BR/A8-35, donc d'une petite taille compatible avec leur utilisation en biochimie et biophysique. Il en est de même pour les complexes tOmpA/télomères non-ioniques, comme cela est clairement apparent sur la Figure 2.

L'innocuité des homotélomères non-ioniques vis-à-vis de la BR est illustrée (Fig. 3) par les spectres UV/visible de la BR piégée en A8-35 ou avec chacun des deux lots d'homotélomère non-ionique testés. Dans les trois cas, le rapport des absorptions à 554 et 280 nm et l'absence de pic significatif à 380 nm indiquent que la protéine est sous sa forme native et n'a pas libéré son cofacteur. (L'absorbance légèrement plus élevée à 280 nm de l'échantillon piégé avec le lot SS174 est due à une légère turbidité).

En résumé, les tests biochimiques effectués permettent d'affirmer que les homotélomères amphiphiles non-ioniques selon l'invention *a*) piègent efficacement les protéines membranaires et les maintiennent en solution en l'absence de détergent ; b) forment avec elles de petits complexes d'une taille et d'une dispersité comparables aux complexes formés avec les amphipols anioniques tels que l'A8-35 ; et c) stabilisent les protéines membranaires par rapport aux solutions détergentes. En d'autres termes, ces polymères possèdent toutes les caractéristiques qui en font des amphipols, et sont susceptibles de se prêter à toutes les applications de ces derniers, avec l'avantage supplémentaire que leur confère leur caractère non-ionique, la reproductibilité élevée de leur synthèse, et la facilité avec laquelle il est possible soit de leur greffer un et un seul groupe fonctionnel déterminé par chaîne télomérique, soit de les fonctionnaliser de façon stochastique comme réalisé précédemment pour l'A8-35.

### RÉFÉRENCES

Prata, C., Giusti, F., Gohon, Y., Pucci, B., Popot, J.-L. & Tribet, C. (2001). Non-ionic amphiphilic polymers derived from Tris(hydroxymethyl)-acrylamidomethane keep membrane proteins soluble and native in the absence of detergent. Biopolymers 56, 77-84.
Sharma, K. S., Durand, G., Giusti, F., Olivier, B., Fabiano, A.-S., Bazzacco, P., Dahmane, T., Ebel, C., Popot, J.-L. & Pucci, B. (2008). Glucose-based amphiphilic telomers designed to keep membrane proteins soluble in aqueous solutions: synthesis and physical-chemical characterization. Langmuir- 24, 13581-13590.
Tribet, C., Audebert, R. & Popot, J.-L. (1996). Amphipols: polymers that keep membrane proteins soluble in aqueous solutions. Proc. Natl. Acad. Sci. USA 93, 15047-15050.
WO 1998/027434
WO 2008/058963

## Revendications

1. Polymère amphiphile comprenant au moins 75% de monomères amphiphiles de formule (I) : 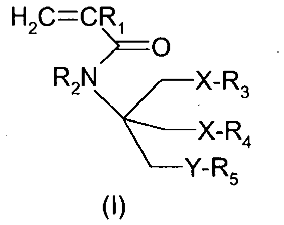 dans laquelle
R₁ et R₂ sont indépendamment choisis parmi H ou un groupe alkyle en C₁-C₆;
X et Y sont indépendamment choisis parmi un atome d'oxygène, un atome de soufre, un groupe carbonyloxy (-(CO)O-) ou oxycarbonyl (-O(CO)-), un groupe uréthane (-OCONH-), et un groupe amide de formule (-CONR₆-) ou (-NR₆CO-) où R₆ est un atome d'hydrogène ou un alkyle en C₁-C₆ ;
R₃ et R₄ sont indépendamment choisis parmi :
a) les groupements glycosidiques,
b) les résidus zwiterioniques,
c) les groupements poly(oxyalkylène) de formule -(O(CH₂)ₓ)_{y}-OH, où x est compris entre 1 et 6, avantageusement x vaut 2, et y est compris entre 4 et 30,
d) les groupements alkylamides de formule -(CH₂)ₙCONR₇R₈ ou -(CH₂)ₙNR₇COR₈ où n est compris entre 1 et 4, R₇ et R₈ sont choisis indépendamment parmi un atome d'hydrogène, un groupe alkyle en C₁-C₆, un groupement glycosidique, un résidu zwiterionique ou un groupement poly(oxyalkylène) de formule -(O(CH₂)ₓ)_{y}-OH, où x et y sont tels que définis ci-dessus,
R₅ est une chaîne hydrocarbonée cyclique (R5 peut contenir un ou deux cycles saturés ou non, notamment de type cyclohexane, cyclopentane ou aromatique) ou acyclique (linéaire ou ramifiée), saturée ou insaturée (une ou plusieurs insaturations) comprenant de 5 à 16 atomes de carbones, ou une chaîne hémifluorocarbonée de formule CₜF₂ₜ₊₁ (CH₂)ₘ avec t compris entre 2 et 10 et m compris entre 2 et 10 ;
la masse molaire moyenne en poids du polymère étant comprise entre 800 et 100 000.

2. Polymère amphiphile selon la revendication 1, **caractérisé en ce que** R₁ et/ou R₂ sont un atome d'hydrogène.

3. Polymère amphiphile selon la revendication 1 ou la revendication 2, **caractérisé en ce que** X est un atome d'oxygène.

4. Polymère amphiphile selon l'une quelconque des revendications 1 à 3, **caractérisé en ce que** Y est un groupe uréthane (-OCONH-).

5. Polymère amphiphile selon l'une quelconque des revendications 1 à 4, **caractérisé en ce que** R₃ et/ou R₄ sont des groupements glycosidiques choisis parmi:
- les mono- ou di-saccharides, notamment les mono- ou di-hexoses de type glucose, mannose, galactose, lactose, allose, altrose, idose, lactose, maltose, et cellobiose , ou
- les mono- ou di-saccharides aminés, tels que la glucosamine, la galactose amine, le fructoseamine, la mannose amine, et l'aminolactitol.

6. Polymère amphiphile selon la revendication 5, **caractérisé en ce que** R₃ et R₄ sont des glucoses.

7. Polymère amphiphile selon l'une quelconque des revendications 1 à 6, **caractérisé en ce que** R₅ est un groupe alkyle en C₅-C₁₆, avantageusement un groupe alkyle linéaire en C₁₁.

8. Polymère amphiphile selon la revendication 1, **caractérisé en ce qu'**il comprend au moins 75% de monomères de formule (III) :

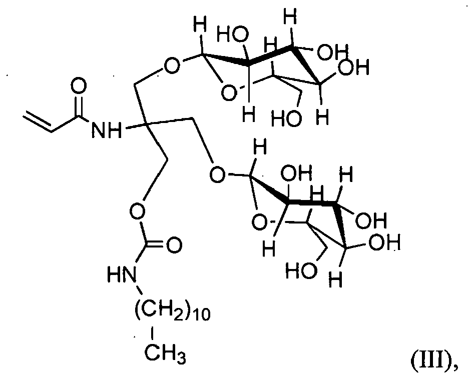

9. Polymère amphiphile selon l'une quelconque des revendications 1-8, **caractérisé en ce qu'**il comprend 100% de monomères amphiphiles de formule (I) ou (III).

10. Polymère amphiphile selon l'une quelconque des revendications 1-9, **caractérisé en ce qu'**il comprend en outre en tête de chaîne un groupement comprenant une fonction thiol de formule R₉-S-, R₉ étant choisi parmi :
- (CH₂)ₘ COOH avec m = 1 à 11,
- (CH₂)ₘ-NH₂ avec m = 2 à 11,
- (CH₂)ₘ-OR₁₀ avec m = 1 à 11 ; X = O, NH, COO, CONH, S, phosphonate P(O)(OR₁₀)₂ ; et R₁₀ choisi parmi H, CH₃, un groupe benzoyle ou benzyle, un agent fluorescent une biotine, un polysaccharide linéaire ou ramifiée comprenant des hexoses, un agent piège à radicaux libres,
- (CH₂)ₘ-CONH(CH₂)ₚS-R₁₁ avec m compris entre 1 et 10, p compris entre 2 et 11, et R₁₁ est choisi parmi H et -C(C₆H₅)₃, un agent fluorescent, un piège à radicaux libre, un oligomère dérivé d'un monomère acrylique ou vinylique.
- (CH₂)ₘ-CO(OCH₂CH₂)ₓOCO(CH₂)ₚS-R₁₁ avec m compris entre 1 et 10, x compris entre 3 et 100, p compris entre 2 et 11, et R₁₁ est choisi parmi H et -C(C₆H₅)₃,
- (CH₂)₂-(-OCH₂CH₂)_{q}-O-R₁₀ avec q = 1 à 100, et R₁₀ est choisi parmi H, CH₃, un groupe benzoyle ou benzyle, un agent fluorescent, une biotine, un monosaccharide ou un polysaccharide linéaire ou branché, éventuellement aminé,
- (CH₂)ᵣCONHC(CH₂OR₁₂)₃, -CH₂CONHC(CH₃)(CH₂OR₁₂)₂, ou CH₂CONHCH(CH₂OR₁₂)₂ avec r compris entre 1 et 11, et R₁₂ est choisi parmi H, un groupe benzyle ou un groupe benzoyle, un agent fluorescent, une biotine, un monosaccharide ou un polysaccharide linéaire ou branché, éventuellement aminé,
- (CH₂)ₘP(O)(OR)₁₃)₂ avec m compris entre 2 et 11, et R₁₃ représente un groupement alkyle de C₁ à C₁₆ linéaire éventuellement substitué,
- une chaîne hydrocarbonée linéaire comprenant 3 à 20 atomes de carbones, saturée ou insaturée, éventuellement substituée, ou
- une chaîne perfluorée de formule CₜF₂ₜ₊₁ (CH₂)ₘ avec t compris entre 2 et 10 et m compris entre 2 et 10.

11. Polymère amphiphile selon la revendication 10, de formule (V) : 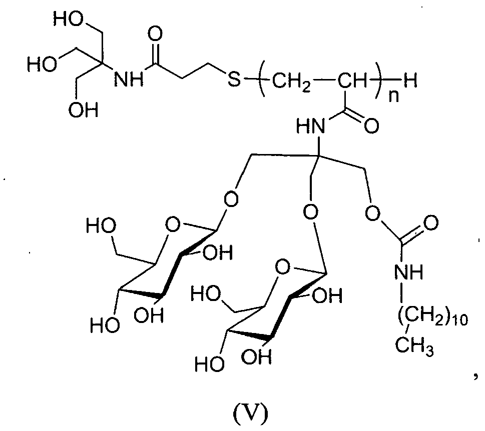 où n est tel que le polymère possède une masse molaire moyenne comprise entre 8000 et 100 000, de préférence entre 8000 et 50000.

12. Procédé de préparation d'un polymère amphiphile selon l'une quelconque des revendications 1-11, comprenant la réaction d'un monomère de formule (I) ou (III) avec un agent de transfert de chaîne en présence d'un initiateur radicalaire dans un solvant anhydre à au moins 60°C.

13. Complexe hydrosoluble d'un composé hydrophobe ou amphiphile, avantageusement une protéine membranaire, et d'un polymère amphiphile selon l'une quelconque des revendications 1-11.

14. Complexe selon la revendication 13, **caractérisé en ce que** la protéine membranaire est choisie dans le groupe constitué par les enzymes membranaires, les récepteurs membranaires, les canaux ioniques membranaires, les antigènes membranaires de microorganismes ou de tumeurs, et les protéines médicaments telles que les anticorps.

15. Complexe selon la revendication 13 ou la revendication 14, sous forme congelée ou lyophilisée.

16. Solution aqueuse possédant une concentration supérieure à 1 g/l, avantageusement entre 10 et 500 g/l d'un ou plusieurs complexe(s) selon l'une quelconque des revendications 13-15.

17. Produit comprenant un support et au moins un complexe selon l'une quelconque des revendications 13-15, ledit complexe étant fixé sur ledit support par l'intermédiaire du polymère amphiphile selon l'une quelconque des revendications 1-10.

18. Utilisation d'un complexe selon l'une quelconque des revendications 13-15, d'une solution aqueuse selon la revendication 16 ou d'un produit selon la revendication 17 pour détecter la présence ou l'absence dans un échantillon biologique d'un ligand dudit composé hydrophobe ou amphiphile.

## Patentansprüche

1. Amiphiles Polymer, umfassend wenigstens 75 % amiphile Monomere der Formel (I): 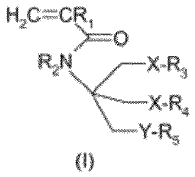 in der
R₁ und R₂ unabhängig voneinander aus H oder einer Alkylgruppe in C₁-C₆ ausgewählt sind;
X und Y unabhängig voneinander aus einem Sauerstoffatom, einem Schwefelatom, einer Carbonyloxy (-(CO)O-) oder Oxycarbonyl (-O(CO)-)-Gruppe, einer Urethangruppe (-OCONH-) und einer Amidgruppe mit der Formel (-CONR₆-) oder (-NR₆CO), in der R6 ein Wasserstoffatom oder ein Alkyl in C₁-C₆ ist, ausgewählt sind;
R₃ und R₄ unabhängig ausgewählt sind aus:
a) den glykosidischen Gruppierungen
b) den zwitterionischen Rückständen,
c) den Poly(-oxyalkylen-)Gruppierungen mit der Formel - (O(CH₂)ₓ)_{y})-OH, in der x zwischen 1 und 6 inbegriffen ist, x vorteilhaft 2 wert ist und y zwischen 4 und 30 inbegriffen ist,
d) den Alkylamid-Gruppierungen mit der Formel - (CH₂)ₙCONR₇R₈ oder - (CH₂)ₙNR₇COR₈, in der n zwischen 1 und 4 inbegriffen ist, R₇ und R₈ unabhängig voneinander aus einem Wasserstoffatom, einer Alkylgruppe in C₁-C₆, einer glykosidischen Gruppe, einem zwitterionischen Rückstand oder einer Poly(-Oxylalkylen-)Gruppierung mit der Formel - (O(CH₂)ₓ)_{y}-OH ausgewählt sind, in der x und y wie oben definiert sind,
R₅ eine zyklische (R₅ kann einen oder zwei saturierte oder nicht saturierte Zyklen, insbesondere vom Typ Cyclohexan, Cyclopentan oder aromatische umfassen) oder azyklische (lineare oder verzweigte), saturierte oder nicht saturierte (eine oder mehrere NichtSättigungen), 5 bis 16 Kohlenstoffatome umfassende, hydrokarbonierte Kette oder eine hemifluorkarbonierte Kette mit der Formel CₜF₂ₜ₊₁ (CH₂)ₘ, wobei t zwischen 2 und 10 inbegriffen ist und m zwischen 2 und 10 inbegriffen ist, ist;
wobei die durchschnittliche molare Gewichtsmasse des Polymers zwischen 800 und 100.000 inbegriffen ist.

2. Amiphiles Polymer gemäß Anspruch 1, **dadurch gekennzeichnet, dass** R₁ und/oder R₂ ein Wasserstoffatom sind.

3. Amiphiles Polymer gemäß Anspruch 1 oder 2, **dadurch gekennzeichnet, dass** X ein Sauerstoffatom ist.

4. Amiphiles Polymer gemäß Anspruch 1 bis 3, **dadurch gekennzeichnet, dass** Y eine Urethan(-OCONH-)- Gruppierung ist.

5. Amiphiles Polymer gemäß Anspruch 1 bis 4, **dadurch gekennzeichnet, dass** R₃ und / oder R₄ glycosidische Gruppierungen sind, die ausgewählt sind aus:
- den Mono- oder Di-Sacchariden, insbesondere den Mono- oder Di-Hexosen vom Typ Glukose, Mannose, Galaktose, Laktose, Allose, Altrose, Idose, Laktose, Maltose und Zellobiose oder
- den aminierten Mono- oder Di-Sacchariden, wie z. B. Glukosamin, Galaktose-Amin, Fruktose-Amin, Mannose-Amin und Aminolaktitol.

6. Amiphiles Polymer gemäß Anspruch 5, **dadurch gekennzeichnet, dass** R₃ und R₄ Glukosen sind.

7. Amiphiles Polymer gemäß Anspruch 1 bis 6, **dadurch gekennzeichnet, dass** R₅ eine Alkylgruppe in C₅-C₁₆, vorteilhaft eine lineare Alkylgruppe in C₁₁ ist.

8. Amiphiles Polymer gemäß Anspruch 1, **dadurch gekennzeichnet, dass** es wenigstens 75 % Monomere der Formel (III) umfasst:

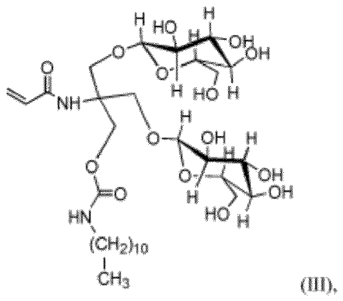

9. Amiphiles Polymer gemäß Anspruch 1 bis 8, **dadurch gekennzeichnet, dass** es 100 % amiphile Monomere mit der Formel (I) oder (III) umfasst.

10. Amiphiles Polymer gemäß Anspruch 1 bis 9, **dadurch gekennzeichnet, dass** es darüber hinaus im Kettenkopf eine Gruppierung umfasst, umfassend eine Thiolfunktion der Formel R₉-S, wobei R₉ ausgewählt ist aus:
- (CH₂)ₘ COOH mit m = 1 bis 11,
- (CH₂)ₘ-NH₂ mit m = 2 bis 11,
- (CH₂)ₘ-OR₁₀ mit m = 1 bis 11; X = O, NH, COO, CONH, S, Phosphanat (P(O) (O-R₁₀)₂; und R₁₀ ausgewählt ist aus H, CH₃, einer Benzoyl oder Benzylgruppe, einem fluoreszierenden Wirkstoff, einem Biotin, einem linearen oder verzweigten Polysaccharid, umfassend Hexosen, einen freien Radikalenfängerwirkstoff,
- (CH₂)ₘ-CONH(CH₂)ₚS-R₁₁, wobei m zwischen 1 und 10 inbegriffen ist, p zwischen 2 und 11 inbegriffen ist und R₁₁ aus H und -C(C₆H₅)₃, einem fluoreszierenden Wirkstoff, einem freien Radikalenfänger, einem aus einem Acryl- oder Vinylmonomer abgeleiteten Oligomer ausgewählt ist.
- (CH₂)ₘ-CO(OCH₂CH₂)ₓOCO(CH₂)ₚS-R₁₁, wobei m zwischen 1 und 10 inbegriffen ist, x zwischen 3 und 100 inbegriffen ist, p zwischen 2 und 11 inbegriffen ist und R₁₁ aus H und -C(C₆H₅)₃ ausgewählt ist,
- (CH₂)₂-(-OCH₂CH₂)_{q}-O-R₁₀ mit q = 1 bis 100, und R₁₀ ist ausgewählt aus H, CH₃, einer Benzoyl- oder Benzylgruppe, einem fluoreszierenden Wirkstoff, einem Biotin, einem Monosaccharid oder einem linearen oder verzweigten, eventuell aminierten Polysaccharid,
- (CH₂)ᵣCONHC(CH₂OR₁₂)₃, -CH₂CONHC(CH₃)(CH₂OR₁₂)₂ oder -CH₂CONHCH(CH₂OR₁₂)₂, wobei r zwischen 1 und 11 inbegriffen ist und R₁₂ aus H, einer Benzyl- oder Benzoylgruppe, einem fluoreszierenden Wirkstoff, einem Biotin, einem Monosaccharid oder einem linearen oder verzweigten, eventuell aminierten Polysaccharid ausgewählt ist,
- (CH₂)ₘP(O)(OR₁₃)₂, wobei m zwischen 2 und 11 inbegriffen ist und R₁₃ eine Alkylgruppierung mit linearem, eventuell substituiertem C₁ bis C₆ darstellt,
- eine hydrogenkarbonierte lineare Kette, umfassend 3 bis 20 gesättigte oder ungesättigte, eventuell substituierte Kohlenstoffatome oder
- eine perfluorierte Kette mit der Formel CₜF₂ₜ₊₁ (CH₂)ₘ, wobei t zwischen 2 und 10 und m zwischen 2 und 10 inbegriffen ist.

11. Amiphiles Polymer gemäß Anspruch 10 mit der Formel (V): 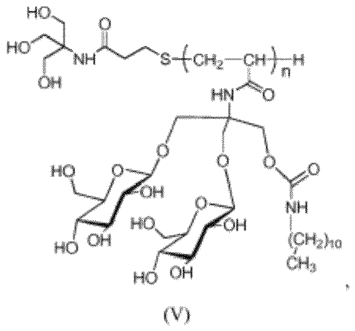 in der n derart ist, dass das Polymer eine zwischen 8.000 und 100.000, bevorzugt zwischen 8.000 und 50.000 inbegriffene durchschnittliche molare Masse hat.

12. Zubereitungsverfahren eines amiphilen Polymers gemäß Anspruch 1 bis 11, umfassend die Reaktion eines Monomers mit der Formel (I) oder (III) mit einem Kettentransfer-Wirkstoff bei Vorhandensein eines Radikaleninitiators in einem wasserfreien Lösungsmittel bei wenigstens 60° C.

13. Wasserlöslicher Komplex einer hydrophoben oder amiphilen Verbindung, vorteilhaft einem Membranprotein und einem amiphilen Polymer gemäß Anspruch 1 bis 11.

14. Komplex gemäß Anspruch 13, **dadurch gekennzeichnet, dass** das Membranprotein aus der Gruppe ausgewählt ist, die aus Membranenzyme, Membranrezeptoren, Membranionenkanäle, Membranantigenen aus Mikroorganismen oder Tumoren und Medikamenten-Proteinen, wie z. B. Antikörpern, ausgewählt ist.

15. Komplex gemäß Anspruch 13 oder Anspruch 14 in tiefgefrorener oder lyophilisierter Form.

16. Wässerige Lösung, die eine Konzentration von mehr als 1 g/l, vorteilhaft zwischen 10 und 500 g/l, einer oder mehrerer Komplex(e) gemäß Anspruch 13 bis 15 besitzt.

17. Produkt, umfassend einen Träger und wenigstens einen Komplex gemäß Anspruch 13 bis 15, wobei der genannte Komplex auf dem genannten Träger über ein amiphiles Polymer gemäß Anspruch 1 bis 10 fixiert ist.

18. Verwendung eines Komplexes gemäß Anspruch 13 bis 15 einer wässerigen Lösung gemäß Anspruch 16 oder eines Produkts gemäß Anspruch 17 für die Detektion des Vorhandenseins oder des Fehlens eines Liganden der genannten hydrophoben oder amiphilen Verbindung in einer biologischen Probe.

## Claims

1. Amphiphilic polymer comprising at least 75% of amphiphilic monomers of formula (I): 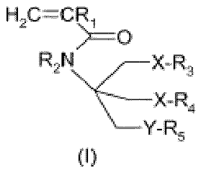 in which
R₁ and R₂ are independently selected from H or a C₁-C₆ alkyl group;
X and Y are independently selected from an oxygen atom, a sulphur atom, a carbonyloxy (-(CO)O-) or oxycarbonyl (-O(CO)-) group, a urethane group(-OCONH-), and an amide group of formula (-CONR₆-) or (-NR₆CO-), wherein R₆ is a hydrogen atom or a C₁-C₆ alkyl;
R₃ and R₄ are independently selected from:
a) glycosidic groups,
b) zwitterionic residues,
c) poly (oxyalkylene) groups of formula -(O(CH₂)ₓ)_{y}-OH, wherein x is comprised between 1 and 6, advantageously x is equal to 2, and y is comprised between 4 and 30,
d) alkyl amide groups of formula -(CH₂)ₙCONR₇R₈- or - (CH2)ₙNR₇COR₈ wherein n is comprised between 1 and 4, R₇ and R₈ are selected independently from a hydrogen atom, a C₁-C₆, alkyl group, a glycosidic group, a zwitterionic residue or a poly(oxyalkylene) group of formula - (O(CH₂)ₓ)_{y}-OH, wherein x and y are as defined above,
R₅ is a cyclic (R₅ may contain one or two cycles, saturated or not, particularly of cyclohexane, cyclopentane or aromatic type) or acyclic (linear or branched), saturated or unsaturated (one or more unsaturations), hydrocarbon chain comprising from 5 to 16 carbon atoms, or a hemifluorocarbonated chain of formula CₜF₂ₜ₊₁ (CH₂)ₘ with t comprised between 2 and 10 and m comprised between 2 and 10;
the average molar mass by weight of the polymer being comprised between 800 and 100 000.

2. Amphiphilic polymer according to claim 1, **characterised in that** R₁ and/or R₂ are a hydrogen atom.

3. Amphiphilic polymer according to claim 1 or claim 2, **characterised in that** X is an oxygen atom.

4. Amphiphilic polymer according to any of claims 1 to 3, **characterised in that** Y is a urethane group (-OCONH-).

5. Amphiphilic polymer according to any of claims 1 to 4, **characterised in that** R₃ and/or R₄ are glycosidic groups selected from:
- mono- or di-saccharides, particularly mono- or di-hexoses of glucose, mannose, galactose, lactose, allose, altrose, idose, lactose, maltose, and cellobiose type, or
- aminated mono- or di-saccharides, such as glucosamine, aminated galactose, fructosamine, aminated mannose, and aminolactitol.

6. Amphiphilic polymer according to claim 5, **characterised in that** R₃ and R₄ are glucoses.

7. Amphiphilic polymer according to any of claims 1 to 6, **characterised in that** R₅ is a C₅-C₁₆ alkyl group, advantageously a C₁₁ linear alkyl group.

8. Amphiphilic polymer according to claim 1, **characterised in that** it comprises at least 75% of monomers of formula (III):

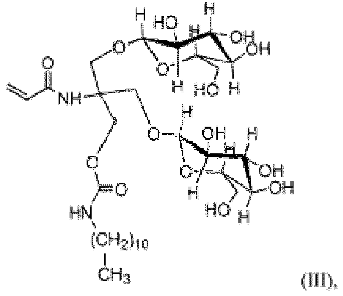

9. Amphiphilic polymer according to any of claims 1-8, **characterised in that** it comprises 100% of amphiphilic monomers of formula (I) or (III).

10. Amphiphilic polymer according to any of claims 1-9, **characterised in that** it further comprises at the head of the chain a group comprising a thiol function of formula R₉-S-, R₉ being selected from:
- (CH₂)ₘ COOH with m = 1 to 11,
- (CH₂)ₘ-NH₂ with m = 2 to 11,
- (CH₂)ₘ-OR₁₀ with m = 1 to 11; X = 0, NH, COO, CONH, S, phosphonate P(O) (O-R₁₀)₂; and R₁₀ selected from H, CH₃, a benzoyl or benzyl group, a fluorescent agent, a biotin, a linear or branched polysaccharide comprising hexoses, a free radical trapping agent,
- (CH₂)ₘ-CONH(CH₂)ₚS-R₁₁ with m comprised between 1 and 10, p comprised between 2 and 11, and R₁₁ is selected from H and -C(C₆H₅)₃, a fluorescent agent, a free radical trap, an oligomer derivative of an acrylic or vinylic monomer.
- (CH₂)ₘ-CO(OCH₂CH₂)ₓOCO(CH₂)ₚS-R₁₁ with m comprised between 1 and 10, x comprised between 3 and 100, p comprised between 2 and 11, and R₁₁ is selected from H and -C(C₆H₅)₃,
- (CH₂)₂-(-OCH₂CH₂)_{q}-O-R₁₀ with q = 1 to 100, and R₁₀ is selected from H, CH₃, a benzoyl or benzyl group, a fluorescent agent, a biotin, a monosaccharide or a linear or branched polysaccharide, optionally aminated,
- (CH₂)ᵣCONHC(CH₂OR₁₂)₃, -CH₂CONHC((CH₃)(CH₂OR₁₂)₂, or CH₂CONHCH(CH₂OR₁₂)₂ with r comprised between 1 and 11, and R₁₂ is selected from H, a benzyl or a benzoyl group, a fluorescent agent, a biotin, a monosaccharide or a polysaccharide, linear or branched, optionally aminated,
- (CH₂)ₘP(O)(OR₁₃)₂ with m comprised between 2 and 11, and R₁₃ represents a C₁ to C₁₆ linear alkyl group, optionally substituted,
- a linear hydrocarbon chain comprising 3 to 20 carbon atoms, saturated or unsaturated, optionally substituted, or
- a perfluorated chain of formula CₜF₂ₜ₊₁ (CH₂)ₘ with t comprised between 2 and 10 and m comprised between 2 and 10.

11. Amphiphilic polymer according to claim 10, of formula (V): 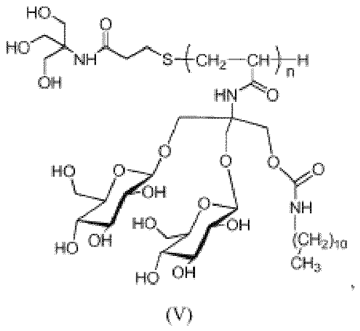 wherein n is such that the polymer has an average molar mass comprised between 8000 and 100000, preferably between 8000 and 50000.

12. Method of preparing an amphiphilic polymer according to any of claims 1-11, comprising the reaction of a monomer of formula (I) or (III) with a chain transfer agent in the presence of a radical initiator in an anhydrous solvent at at least 60 °C.

13. Water soluble complex of a hydrophobic or amphiphilic compound, advantageously a membrane protein, and an amphiphilic polymer according to any of claims 1-11.

14. Complex according to claim 13, **characterised in that** the membrane protein is selected from the group constituted of membrane enzymes, membrane receptors, membrane ion channels, membrane antigens of microorganisms or tumours, and medicinal proteins such as antibodies.

15. Complex according to claim 13 or claim 14, in frozen or lyophilised form.

16. Aqueous solution having a concentration greater than 1 g/l, advantageously between 10 and 500 g/l, of one or more complex (es) according to any of claims 13-15.

17. Product comprising a support and at least one complex according to any of claims 13-15, said complex being fixed on said support through the amphiphilic polymer according to any of claims 1-10.

18. Use of a complex according to any of claims 13-15, of an aqueous solution according to claim 16 or of a product according to claim 17 to detect the presence or the absence in a biological sample of a ligand of said hydrophobic or amphiphilic compound.
